# EUROPEAN PATENT APPLICATION

(11) **EP 4 088 715 A1**
(43) Date of publication of application: **16.11.2022**
(21) Application number: 21173895.0
(22) Date of filing: 14.05.2021
(51) Int. Cl.: A61K 9/20, A61K 31/216, A61K 31/41

(54) **PHARMACEUTICAL FORMULATION OF VALSARTAN AND SACUBITRIL**

(71) Applicant: KRKA, d.d., Novo mesto, 8501 Novo mesto (SI)
(72) Inventor: Benkovic, Marko, 8501 Novo mesto (SI); Klement, Dejan, 8501 Novo mesto (SI); Meznar, Klavdija, 8501 Novo mesto (SI); Podgorsek, Katja, 8501 Novo mesto (SI); Benkovic, Marko, 8501 Novo mesto (SI); Korasa, Klemen, 8501 Novo mesto (SI); Smrkolj, Matej, 8501 Novo mesto (SI)
(74) Representative: Kutzenberger Wolff & Partner

(57) **Abstract**

The invention relates to a pharmaceutical formulation comprising a mixture of a first granular composition comprising valsartan disodium salt and a second composition comprising or essentially consisting of sacubitril sodium salt. The second composition may be non-granular or granular. The invention further relates to a pharmaceutical dosage form comprising such pharmaceutical formulation and to a process for preparing such pharmaceutical formulation.

## Description

The invention relates to a pharmaceutical formulation comprising a mixture of a first granular composition comprising valsartan disodium salt and a second composition comprising or essentially consisting of sacubitril sodium salt. The second composition may be non-granular or granular. The invention further relates to a pharmaceutical dosage form comprising such pharmaceutical formulation and to a process for preparing such pharmaceutical formulation.

Pharmaceutical combinations comprising valsartan and sacubitril are known from the prior art. The originator product Entresto^{®} marketed by Novartis contains a co-crystal complex of the sodium salts of sacubitril and valsartan. Entresto^{®} is supplied for oral administration as immediate release film-coated tablets in three strengths: 24/26, 49/51 and 97/103, containing 24.3 mg sacubitril/25.7 mg valsartan, 48.6 mg sacubitril/51.4 mg valsartan, and 97.2 mg sacubitril/102.8 mg valsartan, respectively. According to the information published by the EMA in the European Public Assessment Report (EPAR), this combination leads to a higher valsartan bioavailability compared to formulations comprising only valsartan or valsartan in other combinations.

Various dosage forms comprising valsartan and sacubitril or their pharmaceutically acceptable salts in amorphous or crystalline form with one or more pharmaceutically acceptable excipients are known from the prior art. In this regard it can be referred to e.g. WO 2017 042 700 A1, WO 2017 036 420 A1, WO 2017 020 841 A1, WO 2017 012 917 A1, WO 2017 009 784 A1, and WO 2017 000 864 A1.

Among such dosage forms, dosage forms comprising valsartan and sacubitril as two separate active compounds in separate entities are also known from the prior art, e.g. from WO 2017 012 600 A1, WO 2019 008 485 A1, WO 2019 020 706 A1, and EP 3 766 484 A1.

The pharmaceutical formulations comprising valsartan and sacubitril of the prior art and the methods for their preparation are not satisfactory in every respect and there is a demand for improved pharmaceutical formulations.

When pharmaceutical formulations comprise sacubitril and valsartan in the same phase, the physical and chemical stability of both active ingredients can be reduced, e.g. due to the formation of the trisodium salt of sacubitril and valsartan.

When pharmaceutical formulations comprising sacubitril and valsartan are made by methods involving dry granulation, the production process is highly dependent on the materials entering the granulation. For example, a difference in the particle size of the active pharmaceutical ingredient (API) can lead to problems during roller compaction due to poor flowability.

It is an object of the invention to provide pharmaceutical formulations comprising valsartan and sacubitril that have advantages compared to the prior art. The pharmaceutical formulations should have improved stability and flow properties thereby facilitating processability of tableting mixtures. Further it is an object of the invention to provide pharmaceutical dosage forms comprising valsartan and sacubitril that have advantages compared to the prior art. The pharmaceutical dosage forms should provide robust and fast dissolution profiles and should be preparable in an easy manner.

This object has been achieved by the subject-matter of the patent claims.

It has been surprisingly found that advantageous pharmaceutical formulations can be prepared comprising a first granular composition and a second composition. The first granular composition comprises valsartan disodium salt, preferably in an amorphous form. The second composition comprises sacubitril sodium salt, preferably in a crystalline form. The second composition may be non-granular or granular. It has been surprisingly found that such granulated pharmaceutical formulations exhibit improved stability compared to pharmaceutical formulations comprising both active ingredients in the same phase. It has been surprisingly found that both active ingredients remain in the desired form throughout their shelf life.

Further, it has been surprisingly found that such granulated pharmaceutical formulations can be advantageously prepared by wet granulation. When preparing the first granular composition comprising the valsartan disodium salt by wet granulation, improved flow characteristics of the granulate can be achieved compared to dry granulation. It has been surprisingly found that wet granulation, e.g. fluid bed granulation, with valsartan disodium salt dissolved in the granulation fluid leads to a faster flow time and a lower repose angle compared to dry granulation of valsartan disodium salt.

Still further, it has been surprisingly found that when the valsartan disodium salt is dissolved in the granulation liquid that is used for wet granulation, a robust granulation process is provided. Under these wet granulation conditions, the particle size of valsartan entering the granulation has no influence on the granulation process and the size of the granules can be predicted and controlled in an easy manner. Further, the dissolution profiles of dosage forms made from such granules are robust.

Yet further, it has been surprisingly found that valsartan disodium salt can be advantageously granulated from aqueous granulation liquids, i.e. organic solvents can be avoided which would otherwise require specific granulation equipment and security measures.

Furthermore, it has been surprisingly found that sacubitril sodium can be added during the preparation of the pharmaceutical formulation (tableting mixture) as a second composition, because the flow properties of sacubitril sodium are adequate for enabling a good level of processability. The second composition may be non-granular or granular, and when the second composition is a second granular composition, it may be dry granulated, hot-melt granulated or wet granulated.

A first aspect of the invention relates to a pharmaceutical formulation comprising a mixture of
- a first granular composition comprising valsartan disodium salt; and
- a second composition comprising or essentially consisting of sacubitril sodium salt. The second composition may be non-granular or granular.

For the purpose of the specification, unless expressly stated otherwise, *"essentially consisting of"* or *"essentially the total amount"* preferably refers to a content of at least 95 wt.-%, more preferably at least 98 wt.-%, still more preferably at least 99.0 wt.-%, yet more preferably at least 99.90 wt.-%, and in particular 100 wt.-%.

Unless expressly stated otherwise, all references to Ph. Eur. refer to the 10th edition in the version valid on May 1, 2021.

In preferred embodiments, the pharmaceutical formulation according to the invention is a solid mixture comprising
- the first granular composition (intragranular phase); and
- the second composition which is non-granular (extragranular phase).

While it is contemplated that the first granular composition may be prepared by dry granulation, hot-melt granulation or wet granulation, preferably the first granular composition has been obtained by wet granulation (see Examples 1 to 7).

In other preferred embodiments, the pharmaceutical formulation according to the invention is a solid mixture comprising
- the first granular composition (first intragranular phase); and
- the second composition which is granular as well, i.e. a second granular composition (second intragranular phase).

In still other preferred embodiments, the pharmaceutical formulation according to the invention is a solid mixture comprising
- the first granular composition (first intragranular phase);
- the second composition which is granular as well, i.e. a second granular composition (second intragranular phase); and
- a third composition which is preferably non-granular (extragranular phase).

In preferred embodiments, the second granular composition has been obtained by dry granulation or hot-melt granulation, preferably by dry granulation, e.g. roller compaction (see Example 8).

In other preferred embodiments, the second granular composition has been obtained by wet granulation, e.g. fluidized bed granulation (see Examples 9 and 10).

In preferred embodiments, the pharmaceutical formulation according to the invention essentially consists of the first granular composition and the second composition.

In other preferred embodiments, the pharmaceutical formulation according to the invention essentially consists of the first granular composition, the second composition which is granular as well (i.e. second granular composition), and the third composition. Preferably, the third composition comprises neither valsartan disodium salt nor sacubitril sodium salt.

The pharmaceutical formulation according to the invention can preferably be regarded as an intermediate product, e.g. a tableting mixture, useful for the preparation of compressed or compacted pharmaceutical dosage forms, particularly for the preparation of tablets.

The pharmaceutical formulation according to the invention is a mixture. The mixture comprises the first granular composition and the second composition. The mixture is typically a homogenous mixture, where the granules of the first granular mixture are intimately mixed with the constituents of the second composition, and optionally with a third composition. When the second composition is a second granular compositions, the granules of the first granular composition are mixed with the granules of the second granular composition, and optionally with a third composition. When the pharmaceutical formulation according to the invention, i.e. the mixture comprising the first granular composition and the second composition, is compressed or compacted into tablets, the tablets thus obtained contain the first granular composition and the second composition in admixture with one another. In other words, compression or compaction does not provide e.g. layer tablets where the first granular composition forms one layer and the second composition forms another separate layer.

Preferably, at least a portion of the valsartan disodium salt is present in amorphous form.

Preferably, at least 80 wt.-% of the valsartan disodium salt that is comprised in the formulation is present in amorphous form; preferably at least 85 wt.-%, more preferably at least 90 wt.-%, and in particular essentially the total amount.

Preferably, essentially the total amount of the valsartan disodium salt that is comprised in the formulation is present in the first granular composition.

In preferred embodiments of the formulation, the weight content of valsartan disodium salt relative to the total weight of the formulation is at least 7.0 wt.-%, preferably at least 10 wt.-%, more preferably at least 13 wt.-%, still more preferably at least 16 wt.-%, yet more preferably at least 19 wt.-%, even more preferably at least 22 wt.-%, most preferably at least 25 wt.-%, and in particular at least 28 wt.-%.

In preferred embodiments of the formulation, the weight content of valsartan disodium salt relative to the total weight of the formulation is at most 60 wt.-%, preferably at most 55 wt.-%, more preferably at most 50 wt.-%, still more preferably at most 45 wt.-%, yet more preferably at most 40 wt.-%, even more preferably at most 35 wt.-%, most preferably at most 30 wt.-%, and in particular at most 25 wt.-%.

In preferred embodiments of the formulation, the weight content of valsartan disodium salt relative to the total weight of the formulation is within the range of from 20±2.0 wt.-%, or 22±4.0 wt.-%, or 22±2.0 wt.-%, or 24±6.0 wt.-%, or 24±4.0 wt.-%, or 24±2.0 wt.-%, or 26±8.0 wt.-%, or 26±6.0 wt.-%, or 26±4.0 wt.-%, or 26±2.0 wt.-%, or 30±10 wt.-%, or 30±8.0 wt.-%, or 30±6.0 wt.-%, or 30±4.0 wt.-%, or 30±2.0 wt.-%.

Preferably, besides the valsartan disodium salt no other forms of valsartan are present.

Preferably, at least a portion of the sacubitril sodium salt is present in crystalline form.

Preferably, at least 80 wt.-% of the sacubitril sodium salt that is comprised in the formulation is present in crystalline form; preferably at least 85 wt.-%, more preferably at least 90 wt.-%, still more preferably at least 95 wt.-%, yet more preferably at least 98 wt.-%, even more preferably at least 99 wt.-%, and in particular essentially the total amount.

Preferably, essentially the total amount of the sacubitril sodium salt that is comprised in the formulation is present in the second composition, which may be non-granular or granular.

In preferred embodiments of the formulation, the weight content of sacubitril sodium salt relative to the total weight of the formulation is at least 4.0 wt.-%, preferably at least 7.0 wt.-%, more preferably at least 10 wt.-%, still more preferably at least 13 wt.-%, yet more preferably at least 16 wt.-%, even more preferably at least 19 wt.-%, most preferably at least 22 wt.-%, and in particular at least 25 wt.-%.

In preferred embodiments of the formulation, the weight content of sacubitril sodium salt relative to the total weight of the formulation is at most 55 wt.-%, preferably at most 50 wt.-%, more preferably at most 45 wt.-%, still more preferably at most 40 wt.-%, yet more preferably at most 35 wt.-%, even more preferably at most 30 wt.-%, most preferably at most 25 wt.-%, and in particular at most 22 wt.-%.

In preferred embodiments of the formulation, the weight content of sacubitril sodium salt relative to the total weight of the formulation is within the range of from 18±2.0 wt.-%, or 20±4.0 wt.-%, or 20±2.0 wt.-%, or 22±6.0 wt.-%, or 22±4.0 wt.-%, or 22±2.0 wt.-%, or 24±8.0 wt.-%, or 24±6.0 wt.-%, or 24±4.0 wt.-%, or 24±2.0 wt.-%, or 26±10 wt.-%, or 26±8.0 wt.-%, or 26±6.0 wt.-%, or 26±4.0 wt.-%, or 26±2.0 wt.-%.

Preferably, besides sacubitril sodium salt no other forms of sacubitril are present.

In preferred embodiments of the formulation, the weight ratio of the valsartan disodium salt to the sacubitril sodium salt is within the range of from 1.5:1.0 to 1.0:1.5, preferably 1.4:1.0 to 1.0:1.4, more preferably 1.3:1.0 to 1.0:1.3, still more preferably 1.2:1.0 to 1.0:1.2, and yet more preferably 1.1:1.0 to 1.0:1.1.

Preferably, besides valsartan disodium salt and sacubitril sodium salt no other pharmacologically active ingredients are present.

In preferred embodiments of the formulation, the weight content of the first granular composition relative to the total weight of the formulation is at least 40 wt.-%, preferably at least 45 wt.-%, more preferably at least 50 wt.-%, still more preferably at least 55 wt.-%, yet more preferably at least 60 wt.-%, even more preferably at least 65 wt.-%, most preferably at least 69 wt.-%, and in particular at least 73 wt.-%.

In preferred embodiments of the formulation, the weight content of the first granular composition relative to the total weight of the formulation is at most 96 wt.-%, preferably at most 92 wt.-%, more preferably at most 88 wt.-%, still more preferably at most 84 wt.-%, yet more preferably at most 80 wt.-%, even more preferably at most 76 wt.-%, most preferably at most 73 wt.-%, and in particular at most 68 wt.-%.

In preferred embodiments of the formulation, the weight content of the first granular composition relative to the total weight of the formulation is within the range of from 61±2.0 wt.-%, or 66±5.0 wt.-%, or 66±2.0 wt.-%, or 71±10 wt.-%, or 71±5.0 wt.-%, or 71±2.0 wt.-%, or 76±15 wt.-%, or 76±10 wt.-%, or 76±5.0 wt.-%, or 76±2.0 wt.-%.

In preferred embodiments of the formulation, the weight content of the second composition relative to the total weight of the formulation is at least 3.0 wt.-%, preferably at least 7.0 wt.-%, more preferably at least 11 wt.-%, still more preferably at least 15 wt.-%, yet more preferably at least 19 wt.-%, even more preferably at least 23 wt.-%, most preferably at least 27 wt.-%, and in particular at least 32 wt.-%.

In preferred embodiments of the formulation, the weight content of the second composition relative to the total weight of the formulation is at most 60 wt.-%, preferably at most 55 wt.-%, more preferably at most 50 wt.-%, still more preferably at most 45 wt.-%, yet more preferably at most 40 wt.-%, even more preferably at most 35 wt.-%, most preferably at most 31 wt.-%, and in particular at most 27 wt.-%.

In preferred embodiments of the formulation, the weight content of the second composition relative to the total weight of the formulation is within the range of from 23±2.0 wt.-%, or 25±4.0 wt.-%, or 25±2.0 wt.-%, or 27±6.0 wt.-%, or 27±4.0 wt.-%, or 27±2.0 wt.-%, or 29±8.0 wt.-%, or 29±6.0 wt.-%, or 29±4.0 wt.-%, or 29±2.0 wt.-%, or 31±10 wt.-%, or 31±8.0 wt.-%, or 31±6.0 wt.-%, or 31±4.0 wt.-%, or 31±2.0 wt.-%, or 33±12 wt.-%, or 33±10 wt.-%, or 33±8.0 wt.-%, or 33±6.0 wt.-%, or 33±4.0 wt.-%, or 33±2.0 wt.-%.

Preferably, the first granular composition is wet granulated, preferably from an aqueous starting material.

Preferably, when the second composition is a granular composition as well, the second composition may be wet granulated, hot-melt granulated or dry granulated, preferably dry granulated.

Preferably, the first granular composition has a flow time of at most 60 s, preferably at most 55 s, more preferably at most 50 s, still more preferably at most 45 s, yet more preferably at most 40 s, even more preferably at most 35 s, most preferably at most 30 s, and in particular at most 25 s; preferably determined in accordance with Ph. Eur. 2.9.16.

Preferably, the first granular composition has a repose angle of at most 70°, preferably at most 65°, more preferably at most 60°, still more preferably at most 55°, yet more preferably at most 50°, even more preferably at most 45°, most preferably at most 40°, and in particular at most 37°; preferably determined in accordance with Ph. Eur. 2.9.36.

Preferably, the first granular composition has a bulk volume of at least 1.0 ml/g, preferably at least 1.4 ml/g, more preferably at least 1.8 ml/g, still more preferably at least 2.2 ml/g, yet more preferably at least 2.6 ml/g, even more preferably at least 3.0 ml/g, most preferably at least 3.4 ml/g, and in particular at least 3.8 ml/g; preferably determined in accordance with Ph. Eur. 2.9.34.

Preferably, the first granular composition has a tapped volume of at least 0.8 ml/g, preferably at least 1.1 ml/g, more preferably at least 1.4 ml/g, still more preferably at least 1.7 ml/g, yet more preferably at least 2.0 ml/g, even more preferably at least 2.3 ml/g, most preferably at least 2.6 ml/g, and in particular at least 2.9 ml/g; preferably determined in accordance with Ph. Eur. 2.9.34.

In preferred embodiments of the formulation, the first granular composition comprises one or more diluents, one or more binders, one or more disintegrants, one or more glidants, one or more lubricants, and/or mixtures thereof.

Preferably, the first granular composition comprises one or more diluents.

For the purpose of the specification a diluent is considered equivalent to a filler. Thus, both excipients perform the same functions, namely to increase the weight and improve the uniformity of the contents.

In preferred embodiments, the one or more diluents are independently of one another selected from the group consisting of
- mono- or oligosaccharides or their derivatives; preferably selected from lactose, e.g. lactose monohydrate, anhydrous lactose, spray dried and/or granulated lactose; sucrose, fructose, dextrates, saccharose, raffinose, trehalose, fructose, and dextrin;
- sugar alcohols; preferably selected from xylitol, mannitol, maltitol, isomalt, and sorbitol;
- cellulose or cellulose derivatives; preferably powdered cellulose, microcrystalline cellulose, or silicified microcrystalline cellulose;
- starch or starch derivatives; preferably low moisture starch, corn starch, pregelatinized starch, or low moisture pregelatinized starch;
- silicates; preferably magnesium aluminometasilicate such as Neusilin;
- salts of phosphoric acid; preferably calcium salts of phosphoric acid; preferably selected from calcium hydrogen phosphate anhydrous and calcium hydrogen phosphate hydrate;
- salts of carbonic acid; preferably calcium carbonate, sodium carbonate, potassium carbonate, calcium hydrogencarbonate, sodium hydrogencarbonate, or potassium hydrogencarbonate;
- salts of lactic acid; preferably calcium lactate; and
- mixtures thereof;
preferably from microcrystalline cellulose, saccharides (preferably selected from lactose and saccharose), sugar alcohols (preferably mannitol), and mixtures thereof;
more preferably mannitol, microcrystalline cellulose (such as Avicel PH101) and mixtures thereof.

In preferred embodiments, the total weight content of the one or more diluents relative to the total weight of the formulation is at least 4.0 wt.-%, preferably at least 8.0 wt.-%, more preferably at least 12 wt.-%, still more preferably at least 16 wt.-%, yet more preferably at least 20 wt.-%, even more preferably at least 24 wt.-%, most preferably at least 28 wt.-%, and in particular at least 32 wt.-%;

In preferred embodiments, the total weight content of the one or more diluents relative to the total weight of the formulation is at most 50 wt.-%, preferably at most 45 wt.-%, more preferably at most 40 wt.-%, still more preferably at most 35 wt.-%, yet more preferably at most 32 wt.-%, even more preferably at most 28 wt.-%, most preferably at most 24 wt.-%, and in particular at most 20 wt.-%.

In preferred embodiments, the total weight content of the one or more diluents relative to the total weight of the formulation is within the range of from 16±2.0 wt.-%, or 18±4.0 wt.-%, or 18±2.0 wt.-%, or 20±6.0 wt.-%, or 20±4.0 wt.-%, or 20±2.0 wt.-%, or 24±10 wt.-%, or 24±6.0 wt.-%, or 24±4.0 wt.-%, or 24±2.0 wt.-%, or 28±10 wt.-%, or 28±6.0 wt.-%, or 28±4.0 wt.-%, or 28±2.0 wt.-%, or 32±10 wt.-%, or 32±6.0 wt.-%, or 32±4.0 wt.-%, or 32±2.0 wt.-%.

Preferably, the first granular composition comprises one or more binders.

In preferred embodiments, the one or more binders are independently of one another selected from the group consisting of
- cellulose ethers; preferably selected from hydroxyethyl cellulose, hydroxypropyl cellulose, and hydroxypropyl methyl cellulose;
- gelatin;
- polyvinylpyrrolidone; and
- mixtures thereof;
preferably hydroxypropyl cellulose and polyvinylpyrrolidone;
preferably wherein the hydroxypropyl cellulose is not a low substituted hydroxypropyl cellulose.

Polyvinylpyrrolidone (PVP) is the linear polymer of N-vinylpyrrolidone and is for example sold under the name Povidone K30, commercially available from the HARKE Group. PVP is typically used as binder in pharmaceutical tablets.

For the purpose of the specification hydroxypropyl cellulose, which is preferably not a low substituted hydroxypropyl cellulose, may contain from 53.4% to 80.5% of hydroxypropoxy groups, calculated on the dried base. Exemplary hydroxypropyl celluloses are sold under the tradename Klucel^{®}, commercially available from Ashland.

In preferred embodiments, the total weight content of the one or more binders relative to the total weight of the formulation is at least 2.5 wt.-%, preferably at least 3.0 wt.-%, more preferably at least 3.5 wt.-%, still more preferably at least 4.0 wt.-%, yet more preferably at least 4.5 wt.-%, even more preferably at least 5.0 wt.-%, most preferably at least 5.5 wt.-%, and in particular at least 6.0 wt.-%.

In preferred embodiments, the total weight content of the one or more binders relative to the total weight of the formulation is at most 14 wt.-%, preferably at most 12 wt.-%, more preferably at most 10 wt.-%, still more preferably at most 8.0 wt.-%, yet more preferably at most 6.5 wt.-%, even more preferably at most 5.5 wt.-%, most preferably at most 4.5 wt.-%, and in particular at most 3.5 wt.-%.

In preferred embodiments, the total weight content of the one or more binders relative to the total weight of the formulation is within the range of from 3.0±0.5 wt.-%, or 4.0±1.0 wt.-%, or 4.0±0.5 wt.-%, or 5.0±2.0 wt.-%, or 5.0±1.0 wt.-%, or 5.0±0.5 wt.-%, or 6.0±3.0 wt.-%, or 6.0±2.0 wt.-%, or 6.0±1.0 wt.-%, or 6.0±0.5 wt.-%.

Preferably, the first granular composition comprises one or more disintegrants.

In preferred embodiments, the one or more disintegrants are independently of one another selected from the group consisting of
- crospovidone;
- cellulose or cellulose derivatives; preferably microcrystalline cellulose, low substituted hydroxypropyl cellulose, methylcellulose, sodium salts of carboxymethyl cellulose, calcium salts of carboxymethyl cellulose, cross-linked carboxymethylcellulose and salts thereof (e.g. croscarmellose sodium and/or croscarmellose calcium);
- starch and starch derivatives; preferably native starch, pregelatinized starch, sodium starch glycollate, or hydroxypropyl starch;
- heteroglycanes; preferably alginic acid, sodium alginate, calcium alginate, agar, or guar gum;
- glucosamines; preferably chitosan;
- polacrilin potassium or docusate sodium; and
- mixtures thereof;
preferably from microcrystalline cellulose, croscarmellose sodium, low substituted hydroxypropyl cellulose, crospovidone, and mixtures thereof;
more preferably low substituted hydroxypropyl cellulose, crospovidone and mixtures thereof.

In accordance with Ph. Eur. low substituted hydroxypropyl cellulose is O-(2-hydroxypropylated) cellulose containing from 5.0% to 16.0% of hydroxypropoxy groups, calculated on the dried basis.

Crospovidone (polyvinylpolypyrrolidone, PVPP) is a highly crosslinked modification of polyvinylpyrrolidone (PVP) and is typically used as disintegrant in pharmaceutical tablets.

Preferably, the first granular composition comprises low substituted hydroxypropyl cellulose in combination with crospovidone.

In preferred embodiments, the total weight content of the one or more disintegrants in the first granular composition relative to the total weight of the formulation is at least 5.0 wt.-%, preferably at least 6.0 wt.-%, more preferably at least 7.0 wt.-%, still more preferably at least 8.0 wt.-%, yet more preferably at least 9.0 wt.-%, even more preferably at least 10 wt.-%, most preferably at least 11 wt.-%, and in particular at least 13 wt.-%.

In preferred embodiments, the total weight content of the one or more disintegrants in the first granular composition relative to the total weight of the formulation is at most 45 wt.-%, preferably at most 40 wt.-%, more preferably at most 35 wt.-%, still more preferably at most 30 wt.-%, yet more preferably at most 25 wt.-%, even more preferably at most 20 wt.-%, most preferably at most 15 wt.-%, and in particular at most 12 wt.-%.

In preferred embodiments, the total weight content of the one or more disintegrants in the first granular composition relative to the total weight of the formulation is within the range of from 11±2.0 wt.-%, or 12±3.0 wt.-%, or 12±2.0 wt.-%, or 13±4.0 wt.-%, or 13±3.0 wt.-%, or 13±2.0 wt.-%, or 14±5.0 wt.-%, or 14±4.0 wt.-%, or 14±3.0 wt.-%, or 14±2.0 wt.-%.

In preferred embodiments, the first granular composition comprises low substituted hydroxypropyl cellulose.

In preferred embodiments, the total weight content of the low substituted hydroxypropyl cellulose in the first granular composition relative to the total weight of the formulation is at least 6.0 wt.-%, preferably at least 6.5 wt.-%, more preferably at least 7.0 wt.-%, still more preferably at least 7.5 wt.-%, yet more preferably at least 8.0 wt.-%, even more preferably at least 8.5 wt.-%, most preferably at least 9.0 wt.-%, and in particular at least 9.5 wt.-%.

In preferred embodiments, the total weight content of the low substituted hydroxypropyl cellulose in the first granular composition relative to the total weight of the formulation is at most 45 wt.-%, preferably at most 40 wt.-%, more preferably at most 35 wt.-%, still more preferably at most 30 wt.-%, yet more preferably at most 25 wt.-%, even more preferably at most 20 wt.-%, most preferably at most 15 wt.-%, and in particular at most 10 wt.-%.

In preferred embodiments, the total weight content of the low substituted hydroxypropyl cellulose in the first granular composition relative to the total weight of the formulation is within the range of from 8.3±0.5 wt.-%, or 8.8±1.0 wt.-%, or 8.8±0.5 wt.-%, or 9.3±1.5 wt.-%, or 9.3±1.0 wt.-%, or 9.3±0.5 wt.-%, or 9.8±2.0 wt.-%, or 9.8±1.5 wt.-%, or 9.8±1.0 wt.-%, or 9.8±0.5 wt.-%.

In other preferred embodiments, the first granular composition comprises no low substituted hydroxypropyl cellulose.

In preferred embodiments, the first granular composition comprises crospovidone.

In preferred embodiments, the total weight content of the crospovidone in the first granular composition relative to the total weight of the formulation is at least 0.5 wt.-%, preferably at least 1.0 wt.-%, more preferably at least 1.5 wt.-%, still more preferably at least 2.0 wt.-%, yet more preferably at least 2.5 wt.-%, even more preferably at least 3.0 wt.-%, most preferably at least 3.5 wt.-%, and in particular at least 4.0 wt.-%.

In preferred embodiments, the total weight content of the crospovidone in the first granular composition relative to the total weight of the formulation is at most 20 wt.-%, preferably at most 15 wt.-%, more preferably at most 12 wt.-%, still more preferably at most 10 wt.-%, yet more preferably at most 8.0 wt.-%, even more preferably at most 6.0 wt.-%, most preferably at most 5.0 wt.-%, and in particular at most 4.0 wt.-%.

In preferred embodiments, the total weight content of the crospovidone in the first granular composition relative to the total weight of the formulation is within the range of from 1.2±0.5 wt.-%, or 1.7±1.0 wt.-%, or 1.7±0.5 wt.-%, or 2.7±2.0 wt.-%, or 2.7±1.0 wt.-%, or 2.7±0.5 wt.-%, or 3.7±3.0 wt.-%, or 3.7±2.0 wt.-%, or 3.7±1.0 wt.-%, or 3.7±0.5 wt.-%, or 4.7±4.0 wt.-%, or 4.7±3.0 wt.-%, or 4.7±2.0 wt.-%, or 4.7±1.0 wt.-%, or 4.7±0.5 wt.-%.

In other preferred embodiments, the first granular composition comprises no crospovidone.

Preferably, the first granular composition comprises one or more glidants.

In preferred embodiments, the one or more glidants are independently of one another selected from the group consisting of colloidal silica such as Aerosil 200, talc, magnesium trisilicate, and mixtures thereof; preferably colloidal silica, talc and mixtures thereof.

Preferably, the first granular composition comprises talc in combination with colloidal silica.

In preferred embodiments, the total weight content of the one or more glidants relative to the total weight of the formulation is at least 0.8 wt.-%, preferably at least 0.9 wt.-%, more preferably at least 1.0 wt.-%, still more preferably at least 1.1 wt.-%, yet more preferably at least 1.2 wt.-%, even more preferably at least 1.3 wt.-%, most preferably at least 1.4 wt.-%, and in particular at least 1.5 wt.-%.

In preferred embodiments, the total weight content of the one or more glidants relative to the total weight of the formulation is at most 5.5 wt.-%, preferably at most 5.0 wt.-%, more preferably at most 4.5 wt.-%, still more preferably at most 4.0 wt.-%, yet more preferably at most 3.5 wt.-%, even more preferably at most 3.0 wt.-%, most preferably at most 2.5 wt.-%, and in particular at most 2.0 wt.-%.

In preferred embodiments, the total weight content of the one or more glidants relative to the total weight of the formulation is within the range of from 1.0±0.5 wt.-%, or 1.5±1.0 wt.-%, or 1.5±0.5 wt.-%, or 2.5±2.0 wt.-%, or 2.5±1.0 wt.-%, or 2.5±0.5 wt.-%.

Preferably, the first granular composition comprises talc.

In preferred embodiments, the total weight content of the talc relative to the total weight of the formulation is at least 0.3 wt.-%, preferably at least 0.4 wt.-%, more preferably at least 0.5 wt.-%, still more preferably at least 0.6 wt.-%, yet more preferably at least 0.7 wt.-%, even more preferably at least 0.8 wt.-%, most preferably at least 0.9 wt.-%, and in particular at least 1.0 wt.-%.

In preferred embodiments, the total weight content of the talc relative to the total weight of the formulation is at most 2.4 wt.-%, preferably at most 2.2 wt.-%, more preferably at most 2.0 wt.-%, still more preferably at most 1.8 wt.-%, yet more preferably at most 1.6 wt.-%, even more preferably at most 1.4 wt.-%, most preferably at most 1.2 wt.-%, and in particular at most 1.1 wt.-%.

In preferred embodiments, the total weight content of the talc relative to the total weight of the formulation is within the range of from 0.5±0.2 wt.-%, or 1.0±0.5 wt.-%, or 1.0±0.2 wt.-%, or 1.5±1.0 wt.-%, or 1.5±0.5 wt.-%, or 1.5±0.2 wt.-%.

Preferably, the first granular composition comprises colloidal silica.

In preferred embodiments, the total weight content of the colloidal silica relative to the total weight of the formulation is at least 0.15 wt.-%, preferably at least 0.2 wt.-%, more preferably at least 0.25 wt.-%, still more preferably at least 0.3 wt.-%, yet more preferably at least 0.35 wt.-%, even more preferably at least 0.4 wt.-%, most preferably at least 0.45 wt.-%, and in particular at least 0.5 wt.-%.

In preferred embodiments, the total weight content of the colloidal silica relative to the total weight of the formulation is at most 2.0 wt.-%, preferably at most 1.8 wt.-%, more preferably at most 1.6 wt.-%, still more preferably at most 1.4 wt.-%, yet more preferably at most 1.2 wt.-%, even more preferably at most 1.0 wt.-%, most preferably at most 0.8 wt.-%, and in particular at most 0.6 wt.-%.

In preferred embodiments, the total weight content of the colloidal silica relative to the total weight of the formulation is within the range of from 0.3±0.2 wt.-%, or 0.5±0.4 wt.-%, or 0.5±0.2 wt.-%, or 0.7±0.6 wt.-%, or 0.7±0.4 wt.-%, or 0.7±0.2 wt.-%.

In preferred embodiments, the first granular composition comprises or essentially consists of
- valsartan disodium salt;
- optionally, one diluent;
- one binder;
- one or two disintegrants; and
- optionally, one or two glidants.

In preferred embodiments, the first granular composition comprises or essentially consists of
- valsartan disodium salt, preferably with a total weight content of from 22 to 30 wt.-%;
- optionally, one diluent, preferably with a total weight content of from 17 to 34 wt.-%;
- one binder, preferably with a total weight content of from 0.01 to 6.5 wt.-%, preferably 2.5 to 6.5 wt.-%;
- one or two disintegrants, preferably with a total weight content of from 0.01 to 15 wt.-%, more preferably 4.0 to 15 wt.-%; and
- optionally, one or two glidants, preferably with a total weight content of from 0.5 to 2.0 wt.-%;
in each case relative to the total weight of the formulation.

In preferred embodiments, the first granular composition comprises or essentially consists of
- valsartan disodium salt, preferably with a total weight content of from 22 to 30 wt.-%;
- optionally, microcrystalline cellulose or mannitol; preferably with a total weight content of from 17 to 34 wt.-%;
- polyvinylpyrrolidone or hydroxypropyl cellulose (preferably not low substituted hydroxypropyl cellulose); preferably with a total weight content of from 0.01 to 6.5 wt.-%, preferably 2.5 to 6.5 wt.-%;
- low substituted hydroxypropyl cellulose and/or crospovidone; preferably with a total weight content of from 0.01 to 15 wt.-%, preferably 4.0 to 15 wt.-%; and
- optionally, talc and/or colloidal silica; preferably with a total weight content of from 0.5 to 2.0 wt.-%;
in each case relative to the total weight of the formulation.

In preferred embodiments, the second composition comprises one or more diluents, one or more binders, one or more disintegrants, one or more glidants, one or more lubricants, and/or mixtures thereof.

Preferably, the second composition comprises one or more disintegrants.

Preferably, at least one of the one or more disintegrants comprised in the second composition is the same as one of the one or more disintegrants comprised in the first granular composition.

Preferably, the second composition does not comprise hydroxypropyl cellulose (preferably not low substituted).

In preferred embodiments, the second composition comprises crospovidone.

In preferred embodiments, the total weight content of the crospovidone in the second composition relative to the total weight of the formulation is at least 0.5 wt.-%, preferably at least 1.0 wt.-%, more preferably at least 1.5 wt.-%, still more preferably at least 2.0 wt.-%, yet more preferably at least 2.5 wt.-%, even more preferably at least 3.0 wt.-%, most preferably at least 3.5 wt.-%, and in particular at least 4.0 wt.-%.

In preferred embodiments, the total weight content of the crospovidone in the second composition relative to the total weight of the formulation is at most 20 wt.-%, preferably at most 15 wt.-%, more preferably at most 12 wt.-%, still more preferably at most 10 wt.-%, yet more preferably at most 8.0 wt.-%, even more preferably at most 6.0 wt.-%, most preferably at most 5.0 wt.-%, and in particular at most 4.0 wt.-%.

In preferred embodiments, the total weight content of the crospovidone in the second composition relative to the total weight of the formulation is within the range of from 1.2±0.5 wt.-%, or 1.7±1.0 wt.-%, or 1.7±0.5 wt.-%, or 2.7±2.0 wt.-%, or 2.7±1.0 wt.-%, or 2.7±0.5 wt.-%, or 3.7±3.0 wt.-%, or 3.7±2.0 wt.-%, or 3.7±1.0 wt.-%, or 3.7±0.5 wt.-%, or 4.7±4.0 wt.-%, or 4.7±3.0 wt.-%, or 4.7±2.0 wt.-%, or 4.7±1.0 wt.-%, or 4.7±0.5 wt.-%.

In other preferred embodiments, the second composition comprises no crospovidone.

Preferably, the first granular composition and the second composition comprise crospovidone.

In preferred embodiments, the total weight content of the crospovidone in the formulation relative to the total weight of the formulation is at least 2.0 wt.-%, preferably at least 3.0 wt.-%, more preferably at least 4.0 wt.-%, still more preferably at least 5.0 wt.-%, yet more preferably at least 6.0 wt.-%, even more preferably at least 7.0 wt.-%, most preferably at least 8.0 wt.-%, and in particular at least 9.0 wt.-%.

In preferred embodiments, the total weight content of the crospovidone in the formulation relative to the total weight of the formulation is at most 17 wt.-%, preferably at most 15 wt.-%, more preferably at most 13 wt.-%, still more preferably at most 11 wt.-%, yet more preferably at most 9.0 wt.-%, even more preferably at most 7.0 wt.-%, most preferably at most 5.0 wt.-%, and in particular at most 3.0 wt.-%.

In preferred embodiments, the total weight content of the crospovidone in the formulation relative to the total weight of the formulation is within the range of from 2.0±0.5 wt.-%, or 2.5±1.0 wt.-%, or 2.5±0.5 wt.-%, or 3.0±1.5 wt.-%, or 3.0±1.0 wt.-%, or 3.0±0.5 wt.-%, or 5.0±3.5 wt.-%, or 5.0±1.5 wt.-%, or 5.0±1.0 wt.-%, or 5.0±0.5 wt.-%, or 7.0±5.5 wt.-%, or 7.0±3.5 wt.-%, or 7.0±1.5 wt.-%, or 7.0±1.0 wt.-%, or 7.0±0.5 wt.-%, or 9.0±7.5 wt.-%, or 9.0±5.5 wt.-%, or 9.0±3.5 wt.-%, or 9.0±1.5 wt.-%, or 9.0±1.0 wt.-%, or 9.0±0.5 wt.-%.

Preferably, the total amount of crospovidone comprised in the formulation is divided between the first granular composition and the second composition.

In preferred embodiments, the weight ratio of the crospovidone comprised in the first granular composition to the crospovidone comprised in the second composition is within the range of from 3.0:1.0 to 1.0:3.0, preferably 2.5:1.0 to 1.0:2.5, more preferably 2.0:1.0 to 1.0:2.0, still more preferably 1.5:1.0 to 1.0:1.5, and yet more preferably 1.1:1.0 to 1.0:1.1.

Preferably, the second composition comprises one or more lubricants.

In preferred embodiments, the one or more lubricants are independently of one another selected from the group consisting of
- metal salts of C₁₂₋₂₀-fatty acids and derivatives thereof; preferably selected from magnesium stearate, calcium stearate, aluminum stearate, zinc stearate, magnesium palmitate, magnesium oleate, and sodium stearyl fumarate;
- hydrogenated oils; preferably hydrogenated vegetable oil or hydrogenated castor oil;
- talc;
- meads wax;
- spermaceti;
- boric acid;
- macrogols; and
- mixtures thereof;
preferably from magnesium stearate, calcium stearate, talc, sodium stearyl fumarate, and mixtures thereof;
more preferably sodium stearyl fumarate, magnesium stearate, and mixtures thereof.

In preferred embodiments, the total weight content of the one or more lubricants relative to the total weight of the formulation is at least 0.9 wt.-%, preferably at least 1.2 wt.-%, more preferably at least 1.5 wt.-%, still more preferably at least 1.8 wt.-%, yet more preferably at least 2.1 wt.-%, even more preferably at least 2.4 wt.-%, most preferably at least 2.7 wt.-%, and in particular at least 3.0 wt.-%.

In preferred embodiments, the total weight content of the one or more lubricants relative to the total weight of the formulation is at most 10 wt.-%, preferably at most 9.0 wt.-%, more preferably at most 8.0 wt.-%, still more preferably at most 7.0 wt.-%, yet more preferably at most 6.0 wt.-%, even more preferably at most 5.0 wt.-%, most preferably at most 4.0 wt.-%, and in particular at most 3.5 wt.-%.

In preferred embodiments, the total weight content of the one or more lubricants relative to the total weight of the formulation is within the range of from 2.5±0.5 wt.-%, or 3.0±1.0 wt.-%, or 3.0±0.5 wt.-%, or 3.5±2.0 wt.-%, or 3.5±1.0 wt.-%, or 3.5±0.5 wt.-%.

In preferred embodiments, the second composition comprises or essentially consists of
- sacubitril sodium salt;
- one disintegrant; and
- optionally, one lubricant.

In preferred embodiments, the second composition comprises or essentially consists of
- sacubitril sodium salt, preferably with a total weight content of from 20 to 28 wt.-%;
- one disintegrant, preferably with a total weight content of from 1.0 to 5.0 wt.-%; and
- optionally, one lubricant, preferably with a total weight content of from 2.5 to 3.5 wt.-%;
in each case relative to the total weight of the formulation.

In preferred embodiments, the second composition comprises or essentially consists of
- sacubitril sodium salt, preferably with a total weight content of from 20 to 28 wt.-%;
- crospovidone, preferably with a total weight content of from 1.0 to 5.0 wt.-%; and
- optionally, magnesium stearate; preferably with a total weight content of from 2.5 to 3.5 wt.-%;
in each case relative to the total weight of the formulation.

Preferably, the formulation according to the invention does not comprise sodium lauryl sulphate, preferably no surfactant at all.

Preferably, the formulation according to the invention comprises one or more diluents, one or more binders, and one or more glidants; and wherein the one or more diluents, the one or more binders, and the one or more glidants are comprised in the first granular composition.

In preferred embodiments, the second composition does not comprise
- sodium hydrogen carbonate;
- pregelatinized starch; and/or
- colloidal silica.

In preferred embodiments, the first granular composition comprises or essentially consists of
- valsartan disodium salt;
- optionally, one diluent;
- one binder;
- two disintegrants; and
- optionally, two glidants.

In preferred embodiments, the second composition comprises or essentially consists of
- sacubitril sodium salt;
- one disintegrant; and
- optionally, one lubricant.

In preferred embodiments, the mixture of the formulation according to the invention additionally comprises a third composition which comprises neither valsartan disodium salt nor sacubitril sodium salt and which is preferably non-granular.

When the mixture of the formulation according to the invention additionally comprises the third composition, the second composition is preferably a second granular composition, wherein the first granular composition forms a first intragranular phase, the second granular composition forms a second intragranular phase that is admixed with the first intragranular phase, and the third composition forms an extragranular phase.

In preferred embodiments, the third composition comprises one or more diluents, one or more binders, one or more disintegrants, one or more glidants, one or more lubricants, and/or mixtures thereof.

In preferred embodiments, the third composition comprises or essentially consists of a disintegrant. In preferred embodiments, the disintegrant is crospovidone.

In preferred embodiments, the third composition comprises or essentially consists of a lubricant. In preferred embodiments, the lubricant is magnesium stearate.

In preferred embodiments, the formulation according to the invention comprises or essentially consists of
- valsartan disodium salt;
- one diluent;
- one binder;
- three disintegrants;
- two glidants;
- sacubitril sodium salt; and
- one lubricant;
wherein the valsartan disodium salt, the one diluent, the one binder, two of the three disintegrants, and the two glidants are contained in the first granular composition;
wherein the sacubitril sodium salt, one of the three disintegrants, and the one lubricant are contained in the second composition; and
wherein the formulation does not comprise sodium lauryl sulphate, preferably no surfactant at all.

Another aspect of the invention relates to a pharmaceutical dosage form comprising the pharmaceutical formulation according to the invention.

The dosage form is preferably compacted or compressed.

The dosage form is preferably for oral administration.

The dosage form is preferably selected from the group consisting of tablets, minitablets, microtablets, coated tablets, coated minitablets, coated microtablets, pills, powders, lozenges, sachets, soft gelatin capsules, hard gelatin capsules, and suppositories; preferably tablets.

Preferably, the dosage form is a film-coated tablet.

In preferred embodiments of the dosage form, the dose of valsartan disodium salt is at least 12.9 mg, preferably at least 25.7 mg, more preferably at least 38.6 mg, still more preferably at least 51.4 mg, yet more preferably at least 64.3 mg, even more preferably at least 77.1 mg, most preferably at least 90.0 mg, and in particular at least 128.0 mg.

In preferred embodiments of the dosage form, the dose of valsartan disodium salt is at most 128 mg, preferably at most 105 mg, more preferably at most 92 mg, still more preferably at most 79 mg, yet more preferably at most 66 mg, even more preferably at most 53 mg, most preferably at most 40 mg, and in particular at most 27 mg.

In preferred embodiments of the dosage form, the dose of valsartan disodium salt is within the range of from 10 to 200 mg, preferably from 12 to 185 mg, more preferably from 14 to 170 mg, still more preferably from 16 to 155 mg, yet more preferably from 18 to 140 mg, even more preferably from 20 to 125 mg, most preferably from 22 to 110 mg, and in particular from 25 to 128 mg.

In preferred embodiments of the dosage form, the dose of sacubitril sodium salt is at least 12.2 mg, preferably at least 24.3 mg, more preferably at least 36.5 mg, still more preferably at least 48.6 mg, yet more preferably at least 60.8 mg, even more preferably at least 72.9 mg, most preferably at least 85.1 mg, and in particular at least 97.2 mg.

In preferred embodiments of the dosage form, the dose of sacubitril sodium salt is at most 126 mg, preferably at most 103 mg, more preferably at most 90 mg, still more preferably at most 77 mg, yet more preferably at most 64 mg, even more preferably at most 51 mg, most preferably at most 38 mg, and in particular at most 25 mg.

In preferred embodiments of the dosage form, the dose of sacubitril sodium salt is within the range of from 10 to 200 mg, preferably from 12 to 185 mg, more preferably from 14 to 170 mg, still more preferably from 16 to 155 mg, yet more preferably from 18 to 140 mg, even more preferably from 20 to 125 mg, most preferably from 22 to 110 mg, and in particular from 24 to 100 mg.

In preferred embodiments of the dosage form, the dose of valsartan disodium salt, expressed as equivalent weight of the valsartan free acid, and sacubitril sodium salt, expressed as equivalent weight of the sacubitril free acid, is
- 25.7 mg valsartan and 24.3 mg sacubitril
- 51.4 mg valsartan and 48.6 mg sacubitril; or
- 102.8 mg valsartan and 97.2 mg sacubitril.

In preferred embodiments of the dosage form, the weight ratio of the valsartan disodium salt to the sacubitril sodium salt is within the range of from 1.5:1.0 to 1.0:1.5, preferably 1.4:1.0 to 1.0:1.4, more preferably 1.3:1.0 to 1.0:1.3, still more preferably 1.2:1.0 to 1.0:1.2, and yet more preferably 1.1:1.0 to 1.0:1.1.

Still another aspect of the invention relates to the dosage form according to the invention for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supraventricular and ventricular arrhythmias, atrial fibrillation, atrial flutter, detrimental vascular remodeling, myocardial infarction and its sequelae, atherosclerosis, angina (whether unstable or stable), renal insufficiency (diabetic and non-diabetic), heart failure, angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.

Another aspect of the invention relates to a process for the preparation of a pharmaceutical formulation according to the invention, which involves granulation, preferably wet granulation.

Preferably, the first granular composition is prepared by wet granulation.

In preferred embodiments, the second composition is a powder.

In other preferred embodiments, the second composition is a second granular composition prepared by hot-melt granulation or dry granulation, preferably dry granulation.

In further preferred embodiments, the second composition is a second granular composition prepared by wet granulation.

The process according to the invention preferably comprises the steps of:
(a) providing a granulation liquid comprising
   - (i) valsartan disodium salt or (ii) valsartan free acid and sodium hydroxide;
   - one or more solvents; and
   - optionally, one or more binders;
(b) providing a granulation mixture comprising one or more diluents, one or more disintegrants, one or more glidants, and/or one or more binders;
(c) wet granulating the granulation mixture provided in step (b) with the granulation liquid provided in step (a) thereby obtaining a first granular composition; and
(d) mixing the first granular composition obtained in step (c) with a second composition comprising sacubitril sodium; preferably wherein the second composition is non-granular or granular; thereby obtaining a compression mixture which comprises the first granular composition obtained in step (c) and the second composition comprising the sacubitril sodium.

In preferred embodiments, in step (d) the second composition is a non-granular powder mixture. In other preferred embodiments, in step (d) the second composition is a second granular composition, which has preferably been obtained by dry granulation, hot-melt granulation or wet granulation.

In preferred embodiments, especially when in step (d) the second composition is a second granular composition, the first granular composition obtained in step (c) and the second granular composition are mixed with a third composition, which is preferably non-granular and which preferably comprises neither valsartan disodium salt nor sacubitril sodium salt.

In preferred embodiments, in step (d) the second composition is a non-granular composition (powder mixture) that additionally comprises optionally one or more disintegrants, and optionally one or more lubricants, such that the obtained compression mixture comprises the first granular composition obtained in step (c) and the second composition comprising the sacubitril sodium, the optionally present one or more disintegrants, and the optionally present one or more lubricants.

In other preferred embodiments, in step (d) the second composition is a second granular composition that additionally comprises optionally one or more disintegrants, and optionally one or more lubricants, such that the obtained compression mixture comprises the first granular composition obtained in step (c) and the second granular composition comprising the sacubitril sodium, the optionally present one or more disintegrants, and the optionally present one or more lubricants. In preferred embodiments, the second granular composition has been obtained by dry granulation. In other preferred embodiments, the second granular composition has been obtained by hot-melt granulation. In further preferred embodiments, the second granular composition has been obtained by wet granulation.

Preferably, in step (a) valsartan free acid and sodium hydroxide are provided.

In preferred embodiments, step (a) comprises the sub-steps of:
(a-1) dissolving the sodium hydroxide in the one or more solvents thereby obtaining a sodium hydroxide solution; preferably a clear solution; and
(a-2) adding the valsartan free acid to the sodium hydroxide solution obtained in sub-step (a-1) thereby obtaining a sodium hydroxide valsartan solution.

In preferred embodiments, the sodium hydroxide solution obtained in sub-step (a-1) has a pH value of at least 10, preferably at least 10.5, more preferably at least 11, still more preferably at least 11.5, yet more preferably at least 12, even more preferably at least 12.5, most preferably at least 13, and in particular at least 13.5.

In preferred embodiments, the sodium hydroxide valsartan solution obtained in sub-step (a-2) has a pH value of at least 3.5, preferably at least 4.0, more preferably at least 4.5, still more preferably at least 5.0, yet more preferably at least 5.5, even more preferably at least 6.0, most preferably at least 6.5, and in particular at least 7.0.

In preferred embodiments, the sodium hydroxide valsartan solution obtained in sub-step (a-2) has a pH value of at most 13.5, preferably at most 13, more preferably at most 12.5, still more preferably at most 12, yet more preferably at most 11.5, even more preferably at most 11, most preferably at most 10.5, and in particular at most 10.

In preferred embodiments, the sodium hydroxide valsartan solution obtained in sub-step (a-2) has a pH value within the range of from 3.5 to 13.5, preferably from 4.0 to 13, more preferably from 4.5 to 12.5, still more preferably from 5.0 to 12, yet more preferably from 5.5 to 11.5, even more preferably from 6.0 to 11, most preferably from 6.5 to 10.5, and in particular from 7.0 to 10.

In preferred embodiments, the molar ratio of the sodium hydroxide to the valsartan free acid provided in step (a) is within the range of from 3.5:1.0 to 0.5:1.0, preferably 3.0:1.0 to 1.0:1.0, more preferably 2.5:1.0 to 1.5:1.0, still more preferably 2.3:1.0 to 1.7:1.0, yet more preferably 2.1:1.0 to 1.9:1.0, and in particular 2:1.

Preferably, the one or more solvents provided in step (a) comprise water and/or an organic solvent.

Preferably, the one or more solvents provided in step (a) are selected from the group consisting of water, acetone, ethanol, and mixtures thereof.

Preferably, the one or more solvents provided in step (a) is water.

Preferably, the granulation liquid provided in step (a) is a solution; preferably a clear solution.

In preferred embodiments, the one or more binders optionally provided in step (a) are selected from the group consisting of
- cellulose ethers; preferably selected from hydroxyethyl cellulose, hydroxypropyl cellulose, and hydroxypropyl methyl cellulose;
- gelatin;
- polyvinylpyrrolidone; and
- mixtures thereof;
preferably wherein the hydroxypropyl cellulose is not a low substituted hydroxypropyl cellulose; preferably polyvinylpyrrolidone.

In preferred embodiments, the granulation mixture provided in step (b) comprises
- optionally, one diluent selected from the group consisting of
   - mono- or oligosaccharides or their derivatives; preferably selected from lactose, e.g. lactose monohydrate, anhydrous lactose, spray dried and/or granulated lactose; sucrose, fructose, dextrates, saccharose, raffinose, trehalose, fructose, and dextrin;
   - sugar alcohols; preferably selected from xylitol, mannitol, maltitol, isomalt, and sorbitol;
   - cellulose or cellulose derivatives; preferably powdered cellulose, microcrystalline cellulose, or silicified microcrystalline cellulose;
   - starch or starch derivatives; preferably low moisture starch, corn starch, pregelatinized starch, or low moisture pregelatinized starch;
   - silicates; preferably magnesium aluminometasilicate such as Neusilin;
   - salts of phosphoric acid; preferably calcium salts of phosphoric acid; preferably selected from calcium hydrogen phosphate anhydrous and calcium hydrogen phosphate hydrate;
   - salts of carbonic acid; preferably calcium carbonate, sodium carbonate, potassium carbonate, calcium hydrogencarbonate, sodium hydrogencarbonate, or potassium hydrogencarbonate;
   - salts of lactic acid; preferably calcium lactate; and
   - mixtures thereof;
   preferably from microcrystalline cellulose, saccharides (preferably selected from lactose and saccharose), sugar alcohols (preferably mannitol) and mixtures thereof;
   more preferably mannitol, microcrystalline cellulose (such as Avicel PH101) and mixtures thereof;
- one or two disintegrants independently of one another selected from the group consisting of
   - crospovidone;
   - cellulose or cellulose derivatives; preferably microcrystalline cellulose, low substituted hydroxypropyl cellulose, methylcellulose, sodium salts of carboxymethyl cellulose, calcium salts of carboxymethyl cellulose, cross-linked carboxymethylcellulose and salts thereof (e.g. croscarmellose sodium and/or croscarmellose calcium);
   - starch and starch derivatives; preferably native starch, pregelatinized starch, sodium starch glycollate, or hydroxypropyl starch;
   - heteroglycanes; preferably alginic acid, sodium alginate, calcium alginate, agar, or guar gum;
   - glucosamines; preferably chitosan;
   - polacrilin potassium or docusate sodium; and
   - mixtures thereof;
   preferably from microcrystalline cellulose, croscarmellose sodium, low substituted hydroxypropyl cellulose, crospovidone, and mixtures thereof;
   more preferably low substituted hydroxypropyl cellulose, crospovidone and mixtures thereof;
- optionally, one or two glidants independently of one another selected from the group consisting of colloidal silica such as Aerosil 200, talc, magnesium trisilicate, and mixtures thereof; preferably colloidal silica, talc and mixtures thereof; and
- optionally, one binder selected from the group consisting of
   - cellulose ethers; preferably selected from hydroxyethylcellulose, hydroxypropyl cellulose, and hydroxypropyl methylcellulose;
   - gelatin;
   - polyvinylpyrrolidone; and
   - mixtures thereof;
   preferably wherein the hydroxypropyl cellulose is not a low substituted hydroxypropyl cellulose;
   preferably hydroxypropyl cellulose.

In preferred embodiments, the granulation mixture provided in step (b) comprises
- optionally, microcrystalline cellulose or mannitol;
- low substituted hydroxypropyl cellulose and/or crospovidone;
- optionally, talc and/or colloidal silica; and
- optionally, hydroxypropyl cellulose (preferably not low substituted).

Preferably, in one of the steps (a) and (b), but not in both, the one or more binders are provided.

Preferably, in step (c) the wet granulation is performed by means of a granulating device.

Preferably, in step (c) the granulating device is a high shear mixer or a fluid bed dryer.

In preferred embodiments, step (c) comprises the sub-steps of
(c-1) optionally, heating the granulation mixture provided in step (b) to an elevated temperature; preferably to a temperature within the range of from 40-43 °C, more preferably to about 40 °C;
(c-2) spraying the granulation mixture provided in step (b) or the heated granulation mixture obtained in sub-step (c-1) with the granulation liquid provided in step (a) thereby obtaining wet granules; preferably at a temperature within the range of from 25-33 °C; and
(c-3) drying the wet granules obtained in sub-step (c-2) at elevated temperature thereby obtaining the first granular composition; preferably to a temperature within the range of from 40-43 °C.

Preferably, the first granular composition has a loss on drying after 5 minutes at 105±2 °C of at most 7.5 wt.-%, preferably at most 7.0 wt.-%, more preferably at most 6.5 wt.-%, still more preferably at most 6.0 wt.-%, yet more preferably at most 5.5 wt.-%, even more preferably at most 5.0 wt.-%, most preferably at most 4.5 wt.-%, and in particular at most 4.0 wt.-%. The loss on drying is preferably determined in accordance with Ph. Eur. 2.2.32.

Preferably, sacubitril is provided in step (d) in the form of sacubitril sodium salt.

In preferred embodiments, in step (d) the first granular composition is mixed with
- one disintegrant selected from the group consisting of
   - crospovidone;
   - cellulose or cellulose derivatives; preferably microcrystalline cellulose, low substituted hydroxypropyl cellulose, methylcellulose, sodium salts of carboxymethyl cellulose, calcium salts of carboxymethyl cellulose, cross-linked carboxymethylcellulose and salts thereof (e.g. croscarmellose sodium and/or croscarmellose calcium);
   - starch and starch derivatives; preferably native starch, pregelatinized starch, sodium starch glycollate, or hydroxypropyl starch;
   - heteroglycanes; preferably alginic acid, sodium alginate, calcium alginate, agar, or guar gum;
   - glucosamines; preferably chitosan;
   - polacrilin potassium or docusate sodium; and
   - mixtures thereof;
   preferably from microcrystalline cellulose, croscarmellose sodium, low substituted hydroxypropyl cellulose, crospovidone, and mixtures thereof;
   more preferably crospovidone; and
- optionally, one lubricant selected from the group consisting of
   - metal salts of C₁₂₋₂₀-fatty acids and derivatives thereof; preferably selected from magnesium stearate, calcium stearate, aluminum stearate, zinc stearate, magnesium palmitate, magnesium oleate, and sodium stearyl fumarate;
   - hydrogenated oils; preferably hydrogenated vegetable oil or hydrogenated castor oil;
   - talc;
   - meads wax;
   - spermaceti;
   - boric acid;
   - macrogols; and
   - mixtures thereof;
   preferably from magnesium stearate, calcium stearate, talc, sodium stearyl fumarate, and mixtures thereof;
   more preferably sodium stearyl fumarate, magnesium stearate, and mixtures thereof.

Preferably, in step (d) the first granular composition is mixed with
- crospovidone; and
- optionally, magnesium stearate.

In preferred embodiments, step (d) comprises the sub-steps (d) of:
(d-1) mixing the first granular composition obtained in step (c) with sacubitril sodium and optionally one or more disintegrants thereby obtaining a first compression mixture; and
(d-2) optionally, mixing the first compression mixture obtained in sub-step (d-1) with one or more lubricants thereby obtaining a second compression mixture.

Preferably, in step (d) the mixing is performed by means of a mixing device; preferably a container mixer.

Preferably, the second composition is prepared by wet granulation, by hot-melt granulation, or by dry granulation prior to mixing with the first granular composition in step (d).

Another aspect of the invention relates to a pharmaceutical formulation obtainable by the process according to the invention.

Another aspect of the invention relates to a process for the preparation of a pharmaceutical dosage form according to the invention, the process comprising the steps of:
(A) providing a pharmaceutical formulation according to the invention;
(B) compressing the pharmaceutical formulation provided in step (A) by means of a tablet press thereby obtaining a tablet; and
(C) optionally applying a film coating to the tablet obtains in step (B).

Particularly preferred embodiments of the invention are compiled as clauses 1 to 132 hereinafter:
Clause 1: A pharmaceutical formulation comprising a mixture of - a first granular composition comprising valsartan disodium salt; and - a second composition comprising or essentially consisting of sacubitril sodium salt.
Clause 2: The formulation according to clause 1, wherein the second composition is a non-granular composition.
Clause 3: The formulation according to clause 2, wherein the first granular composition forms an intragranular phase and the second non-granular composition forms an extragranular phase.
Clause 4: The formulation according to clause 1, wherein the second composition is a granular composition.
Clause 5: The formulation according to clause 4, wherein the first granular composition forms a first intragranular phase and the second granular composition forms a second intragranular phase.
Clause 6: The formulation according to any of the preceding clauses, wherein the mixture comprises a third composition which comprises neither valsartan disodium salt nor sacubitril sodium salt.
Clause 7: The formulation according to clause 6, wherein the third composition forms an extragranular phase.
Clause 8: The formulation according to any of the preceding clauses, wherein at least a portion of the valsartan disodium salt is present in amorphous form.
Clause 9: The formulation according to any of the preceding clauses, wherein at least 80 wt.-% of the valsartan disodium salt that is comprised in the formulation is present in amorphous form; preferably at least 85 wt.-%, more preferably at least 90 wt.-%, and in particular essentially the total amount.
Clause 10: The formulation according to any of the preceding clauses, wherein essentially the total amount of the valsartan disodium salt that is comprised in the formulation is present in the first granular composition.
Clause 11: The formulation according to any of the preceding clauses, wherein the weight content of valsartan disodium salt is - at least 7.0 wt.-%, preferably at least 10 wt.-%, more preferably at least 13 wt.-%, still more preferably at least 16 wt.-%, yet more preferably at least 19 wt.-%, even more preferably at least 22 wt.-%, most preferably at least 25 wt.-%, and in particular at least 28 wt.-%; - at most 60 wt.-%, preferably at most 55 wt.-%, more preferably at most 50 wt.-%, still more preferably at most 45 wt.-%, yet more preferably at most 40 wt.-%, even more preferably at most 35 wt.-%, most preferably at most 30 wt.-%, and in particular at most 25 wt.-%; and/or - within the range of from 20±2.0 wt.-%, or 22±4.0 wt.-%, or 22±2.0 wt.-%, or 24±6.0 wt.-%, or 24±4.0 wt.-%, or 24±2.0 wt.-%, or 26±8.0 wt.-%, or 26±6.0 wt.-%, or 26±4.0 wt.-%, or 26±2.0 wt.-%, or 30±10 wt.-%, or 30±8.0 wt.-%, or 30±6.0 wt.-%, or 30±4.0 wt.-%, or 30±2.0 wt.-%; in each case relative to the total weight of the formulation.
Clause 12: The formulation according to any of the preceding clauses, wherein besides the valsartan disodium salt no other forms of valsartan are present.
Clause 13: The formulation according to any of the preceding clauses, wherein at least a portion of the sacubitril sodium salt is present in crystalline form.
Clause 14: The formulation according to any of the preceding clauses, wherein at least 80 wt.-% of the sacubitril sodium salt that is comprised in the formulation is present in crystalline form; preferably at least 85 wt.-%, more preferably at least 90 wt.-%, still more preferably at least 95 wt.-%, yet more preferably at least 98 wt.-%, even more preferably at least 99 wt.-%, and in particular essentially the total amount.
Clause 15: The formulation according to any of the preceding clauses, wherein essentially the total amount of the sacubitril sodium salt that is comprised in the formulation is present in the second composition.
Clause 16: The formulation according to any of the preceding clauses, wherein the weight content of sacubitril sodium salt is - at least 4.0 wt.-%, preferably at least 7.0 wt.-%, more preferably at least 10 wt.-%, still more preferably at least 13 wt.-%, yet more preferably at least 16 wt.-%, even more preferably at least 19 wt.-%, most preferably at least 22 wt.-%, and in particular at least 25 wt.-%; - at most 55 wt.-%, preferably at most 50 wt.-%, more preferably at most 45 wt.-%, still more preferably at most 40 wt.-%, yet more preferably at most 35 wt.-%, even more preferably at most 30 wt.-%, most preferably at most 25 wt.-%, and in particular at most 22 wt.-%; and/or - within the range of from 18±2.0 wt.-%, or 20±4.0 wt.-%, or 20±2.0 wt.-%, or 22±6.0 wt.-%, or 22±4.0 wt.-%, or 22±2.0 wt.-%, or 24±8.0 wt.-%, or 24±6.0 wt.-%, or 24±4.0 wt.-%, or 24±2.0 wt.-%, or 26±10 wt.-%, or 26±8.0 wt.-%, or 26±6.0 wt.-%, or 26±4.0 wt.-%, or 26±2.0 wt.-%; in each case relative to the total weight of the formulation.
Clause 17: The formulation according to any of the preceding clauses, wherein besides sacubitril sodium salt no other forms of sacubitril are present.
Clause 18: The formulation according to any of the preceding clauses, wherein the weight ratio of the valsartan disodium salt to the sacubitril sodium salt is within the range of from 1.5:1.0 to 1.0:1.5, preferably 1.4:1.0 to 1.0:1.4, more preferably 1.3:1.0 to 1.0:1.3, still more preferably 1.2:1.0 to 1.0:1.2, and yet more preferably 1.1:1.0 to 1.0:1.1.
Clause 19: The formulation according to any of the preceding clauses, wherein besides valsartan disodium salt and sacubitril sodium salt no other pharmacologically active ingredients are present.
Clause 20: The formulation according to any of the preceding clauses, wherein the weight content of the first granular composition is - at least 40 wt.-%, preferably at least 45 wt.-%, more preferably at least 50 wt.-%, still more preferably at least 55 wt.-%, yet more preferably at least 60 wt.-%, even more preferably at least 65 wt.-%, most preferably at least 69 wt.-%, and in particular at least 73 wt.-%; - at most 96 wt.-%, preferably at most 92 wt.-%, more preferably at most 88 wt.-%, still more preferably at most 84 wt.-%, yet more preferably at most 80 wt.-%, even more preferably at most 76 wt.-%, most preferably at most 73 wt.-%, and in particular at most 68 wt.-%; and/or - within the range of from 61±2.0 wt.-%, or 66±5.0 wt.-%, or 66±2.0 wt.-%, or 71±10 wt.-%, or 71±5.0 wt.-%, or 71±2.0 wt.-%, or 76±15 wt.-%, or 76±10 wt.-%, or 76±5.0 wt.-%, or 76±2.0 wt.-%; in each case relative to the total weight of the formulation.
Clause 21: The formulation according to any of the preceding clauses, wherein the weight content of the second composition is - at least 3.0 wt.-%, preferably at least 7.0 wt.-%, more preferably at least 11 wt.-%, still more preferably at least 15 wt.-%, yet more preferably at least 19 wt.-%, even more preferably at least 23 wt.-%, most preferably at least 27 wt.-%, and in particular at least 32 wt.-%; - at most 60 wt.-%, preferably at most 55 wt.-%, more preferably at most 50 wt.-%, still more preferably at most 45 wt.-%, yet more preferably at most 40 wt.-%, even more preferably at most 35 wt.-%, most preferably at most 31 wt.-%, and in particular at most 27 wt.-%; and/or - within the range of from 23±2.0 wt.-%, or 25±4.0 wt.-%, or 25±2.0 wt.-%, or 27±6.0 wt.-%, or 27±4.0 wt.-%, or 27±2.0 wt.-%, or 29±8.0 wt.-%, or 29±6.0 wt.-%, or 29±4.0 wt.-%, or 29±2.0 wt.-%, or 31±10 wt.-%, or 31±8.0 wt.-%, or 31±6.0 wt.-%, or 31±4.0 wt.-%, or 31±2.0 wt.-%, or 33±12 wt.-%, or 33±10 wt.-%, or 33±8.0 wt.-%, or 33±6.0 wt.-%, or 33±4.0 wt.-%, or 33±2.0 wt.-%; in each case relative to the total weight of the formulation.
Clause 22: The formulation according to any of the preceding clauses, wherein the first granular composition is wet granulated, preferably from an aqueous starting material.
Clause 23: The formulation according to any of the preceding clauses, wherein the first granular composition comprises one or more diluents, one or more binders, one or more disintegrants, one or more glidants, one or more lubricants, and/or mixtures thereof.
Clause 24: The formulation according to any of the preceding clauses, wherein the first granular composition comprises one or more diluents.
Clause 25: The formulation according to clause 23 or 24, wherein the one or more diluents are independently of one another selected from the group consisting of - mono- or oligosaccharides or their derivatives; preferably selected from lactose, e.g. lactose monohydrate, anhydrous lactose, spray dried and/or granulated lactose; sucrose, fructose, dextrates, saccharose, raffinose, trehalose, fructose, and dextrin; - sugar alcohols; preferably selected from xylitol, mannitol, maltitol, isomalt, and sorbitol; - cellulose or cellulose derivatives; preferably powdered cellulose, microcrystalline cellulose, or silicified microcrystalline cellulose; - starch or starch derivatives; preferably low moisture starch, corn starch, pregelatinized starch, or low moisture pregelatinized starch; - silicates; preferably magnesium aluminometasilicate; - salts of phosphoric acid; preferably calcium salts of phosphoric acid; preferably selected from calcium hydrogen phosphate anhydrous and calcium hydrogen phosphate hydrate; - salts of carbonic acid; preferably calcium carbonate, sodium carbonate, potassium carbonate, calcium hydrogencarbonate, sodium hydrogencarbonate, or potassium hydrogencarbonate; - salts of lactic acid; preferably calcium lactate; and - mixtures thereof; preferably from microcrystalline cellulose, saccharides (preferably selected from lactose and saccharose), sugar alcohols (preferably mannitol), and mixtures thereof; more preferably mannitol, microcrystalline cellulose and mixtures thereof.
Clause 26: The formulation according to any of clauses 23 to 25, wherein the total weight content of the one or more diluents is - at least 4.0 wt.-%, preferably at least 8.0 wt.-%, more preferably at least 12 wt.-%, still more preferably at least 16 wt.-%, yet more preferably at least 20 wt.-%, even more preferably at least 24 wt.-%, most preferably at least 28 wt.-%, and in particular at least 32 wt.-%; - at most 50 wt.-%, preferably at most 45 wt.-%, more preferably at most 40 wt.-%, still more preferably at most 35 wt.-%, yet more preferably at most 32 wt.-%, even more preferably at most 28 wt.-%, most preferably at most 24 wt.-%, and in particular at most 20 wt.-%; and/or - within the range of from 16±2.0 wt.-%, or 18±4.0 wt.-%, or 18±2.0 wt.-%, or 20±6.0 wt.-%, or 20±4.0 wt.-%, or 20±2.0 wt.-%, or 24±10 wt.-%, or 24±6.0 wt.-%, or 24±4.0 wt.-%, or 24±2.0 wt.-%, or 28±10 wt.-%, or 28±6.0 wt.-%, or 28±4.0 wt.-%, or 28±2.0 wt.-%, or 32±10 wt.-%, or 32±6.0 wt.-%, or 32±4.0 wt.-%, or 32±2.0 wt.-%; in each case relative to the total weight of the formulation.
Clause 27: The formulation according to any of the preceding clauses, wherein the first granular composition comprises one or more binders.
Clause 28: The formulation according to clause 27, wherein the one or more binders are independently of one another selected from the group consisting of - cellulose ethers; preferably selected from hydroxyethyl cellulose, hydroxypropyl cellulose, and hydroxypropyl methyl cellulose; - gelatin; - polyvinylpyrrolidone; and - mixtures thereof; preferably hydroxypropyl cellulose and polyvinylpyrrolidone; preferably wherein the hydroxypropyl cellulose is not a low substituted hydroxypropyl cellulose.
Clause 29: The formulation according to clause 27 or 28, wherein the total weight content of the one or more binders is - at least 2.5 wt.-%, preferably at least 3.0 wt.-%, more preferably at least 3.5 wt.-%, still more preferably at least 4.0 wt.-%, yet more preferably at least 4.5 wt.-%, even more preferably at least 5.0 wt.-%, most preferably at least 5.5 wt.-%, and in particular at least 6.0 wt.-%; - at most 14 wt.-%, preferably at most 12 wt.-%, more preferably at most 10 wt.-%, still more preferably at most 8.0 wt.-%, yet more preferably at most 6.5 wt.-%, even more preferably at most 5.5 wt.-%, most preferably at most 4.5 wt.-%, and in particular at most 3.5 wt.-%; and/or - within the range of from 3.0±0.5 wt.-%, or 4.0±1.0 wt.-%, or 4.0±0.5 wt.-%, or 5.0±2.0 wt.-%, or 5.0±1.0 wt.-%, or 5.0±0.5 wt.-%, or 6.0±3.0 wt.-%, or 6.0±2.0 wt.-%, or 6.0±1.0 wt.-%, or 6.0±0.5 wt.-%; in each case relative to the total weight of the formulation.
Clause 30: The formulation according to any of the preceding clauses, wherein the first granular composition comprises one or more disintegrants.
Clause 31: The formulation according to clause 30, wherein the one or more disintegrants are independently of one another selected from the group consisting of - crospovidone; - cellulose or cellulose derivatives; preferably microcrystalline cellulose, low substituted hydroxypropyl cellulose, methylcellulose, sodium salts of carboxymethyl cellulose, calcium salts of carboxymethyl cellulose, cross-linked carboxymethylcellulose and salts thereof (e.g. croscarmellose sodium and/or croscarmellose calcium); - starch and starch derivatives; preferably native starch, pregelatinized starch, sodium starch glycollate, or hydroxypropyl starch; - heteroglycanes; preferably alginic acid, sodium alginate, calcium alginate, agar, or guar gum; - glucosamines; preferably chitosan; - polacrilin potassium or docusate sodium; and - mixtures thereof; preferably from microcrystalline cellulose, croscarmellose sodium, low substituted hydroxypropyl cellulose, crospovidone, and mixtures thereof; more preferably low substituted hydroxypropyl cellulose, crospovidone and mixtures thereof.
Clause 32: The formulation according to any of the preceding clauses, wherein the first granular composition comprises low substituted hydroxypropyl cellulose in combination with crospovidone.
Clause 33: The formulation according to any of clauses 30 to 32, wherein the total weight content of the one or more disintegrants in the first granular composition is - at least 5.0 wt.-%, preferably at least 6.0 wt.-%, more preferably at least 7.0 wt.-%, still more preferably at least 8.0 wt.-%, yet more preferably at least 9.0 wt.-%, even more preferably at least 10 wt.-%, most preferably at least 11 wt.-%, and in particular at least 13 wt.-%; - at most 45 wt.-%, preferably at most 40 wt.-%, more preferably at most 35 wt.-%, still more preferably at most 30 wt.-%, yet more preferably at most 25 wt.-%, even more preferably at most 20 wt.-%, most preferably at most 15 wt.-%, and in particular at most 12 wt.-%; and/or - within the range of from 11±2.0 wt.-%, or 12=L3.0 wt.-%, or 12=L2.0 wt.-%, or 13=L4.0 wt.-%, or 13±3.0 wt.-%, or 13±2.0 wt.-%, or 14±5.0 wt.-%, or 14±4.0 wt.-%, or 14±3.0 wt.-%, or 14±2.0 wt.-%; in each case relative to the total weight of the formulation.
Clause 34: The formulation according to any of the preceding clauses, wherein the first granular composition comprises low substituted hydroxypropyl cellulose.
Clause 35: The formulation according to of clause 34, wherein the total weight content of the low substituted hydroxypropyl cellulose in the first granular composition is - at least 6.0 wt.-%, preferably at least 6.5 wt.-%, more preferably at least 7.0 wt.-%, still more preferably at least 7.5 wt.-%, yet more preferably at least 8.0 wt.-%, even more preferably at least 8.5 wt.-%, most preferably at least 9.0 wt.-%, and in particular at least 9.5 wt.-%; - at most 45 wt.-%, preferably at most 40 wt.-%, more preferably at most 35 wt.-%, still more preferably at most 30 wt.-%, yet more preferably at most 25 wt.-%, even more preferably at most 20 wt.-%, most preferably at most 15 wt.-%, and in particular at most 10 wt.-%; and/or - within the range of from 8.3±0.5 wt.-%, or 8.8±1.0 wt.-%, or 8.8±0.5 wt.-%, or 9.3±1.5 wt.-%, or 9.3±1.0 wt.-%, or 9.3±0.5 wt.-%, or 9.8±2.0 wt.-%, or 9.8±1.5 wt.-%, or 9.8±1.0 wt.-%, or 9.8±0.5 wt.-%; in each case relative to the total weight of the formulation.
Clause 36: The formulation according to any of the preceding clauses, wherein the first granular composition comprises crospovidone.
Clause 37: The formulation according to clause 36, wherein the total weight content of the crospovidone in the first granular composition is - at least 0.5 wt.-%, preferably at least 1.0 wt.-%, more preferably at least 1.5 wt.-%, still more preferably at least 2.0 wt.-%, yet more preferably at least 2.5 wt.-%, even more preferably at least 3.0 wt.-%, most preferably at least 3.5 wt.-%, and in particular at least 4.0 wt.-%; - at most 20 wt.-%, preferably at most 15 wt.-%, more preferably at most 12 wt.-%, still more preferably at most 10 wt.-%, yet more preferably at most 8.0 wt.-%, even more preferably at most 6.0 wt.-%, most preferably at most 5.0 wt.-%, and in particular at most 4.0 wt.-%; and/or - within the range of from 1.2±0.5 wt.-%, or 1.7±1.0 wt.-%, or 1.7±0.5 wt.-%, or 2.7±2.0 wt.-%, or 2.7±1.0 wt.-%, or 2.7±0.5 wt.-%, or 3.7±3.0 wt.-%, or 3.7±2.0 wt.-%, or 3.7±1.0 wt.-%, or 3.7±0.5 wt.-%, or 4.7±4.0 wt.-%, or 4.7±3.0 wt.-%, or 4.7±2.0 wt.-%, or 4.7±1.0 wt.-%, or 4.7±0.5 wt.-%; in each case relative to the total weight of the formulation.
Clause 38: The formulation according to any of the preceding clauses, wherein the first granular composition comprises one or more glidants.
Clause 39: The formulation according to clause 38, wherein the one or more glidants are independently of one another selected from the group consisting of colloidal silica, talc, magnesium trisilicate, and mixtures thereof; preferably colloidal silica, talc and mixtures thereof.
Clause 40: The formulation according to any of the preceding clauses, wherein the first granular composition comprises talc in combination with colloidal silica.
Clause 41: The formulation according to any of clauses 38 to 40, wherein the total weight content of the one or more glidants is - at least 0.8 wt.-%, preferably at least 0.9 wt.-%, more preferably at least 1.0 wt.-%, still more preferably at least 1.1 wt.-%, yet more preferably at least 1.2 wt.-%, even more preferably at least 1.3 wt.-%, most preferably at least 1.4 wt.-%, and in particular at least 1.5 wt.-%; - at most 5.5 wt.-%, preferably at most 5.0 wt.-%, more preferably at most 4.5 wt.-%, still more preferably at most 4.0 wt.-%, yet more preferably at most 3.5 wt.-%, even more preferably at most 3.0 wt.-%, most preferably at most 2.5 wt.-%, and in particular at most 2.0 wt.-%; and/or - within the range of from 1.0±0.5 wt.-%, or 1.5±1.0 wt.-%, or 1.5±0.5 wt.-%, or 2.5±2.0 wt.-%, or 2.5±1.0 wt.-%, or 2.5±0.5 wt.-%; in each case relative to the total weight of the formulation.
Clause 42: The formulation according to any of the preceding clauses, wherein the first granular composition comprises talc.
Clause 43: The formulation according to clause 42, wherein the total weight content of the talc is - at least 0.3 wt.-%, preferably at least 0.4 wt.-%, more preferably at least 0.5 wt.-%, still more preferably at least 0.6 wt.-%, yet more preferably at least 0.7 wt.-%, even more preferably at least 0.8 wt.-%, most preferably at least 0.9 wt.-%, and in particular at least 1.0 wt.-%; - at most 2.4 wt.-%, preferably at most 2.2 wt.-%, more preferably at most 2.0 wt.-%, still more preferably at most 1.8 wt.-%, yet more preferably at most 1.6 wt.-%, even more preferably at most 1.4 wt.-%, most preferably at most 1.2 wt.-%, and in particular at most 1.1 wt.-%; and/or - within the range of from 0.5±0.2 wt.-%, or 1.0±0.5 wt.-%, or 1.0±0.2 wt.-%, or 1.5±1.0 wt.-%, or 1.5±0.5 wt.-%, or 1.5±0.2 wt.-%; in each case relative to the total weight of the formulation.
Clause 44: The formulation according to any of the preceding clauses, wherein the first granular composition comprises colloidal silica.
Clause 45: The formulation according to clause 44, wherein the total weight content of the colloidal silica is - at least 0.15 wt.-%, preferably at least 0.2 wt.-%, more preferably at least 0.25 wt.-%, still more preferably at least 0.3 wt.-%, yet more preferably at least 0.35 wt.-%, even more preferably at least 0.4 wt.-%, most preferably at least 0.45 wt.-%, and in particular at least 0.5 wt.-%; - at most 2.0 wt.-%, preferably at most 1.8 wt.-%, more preferably at most 1.6 wt.-%, still more preferably at most 1.4 wt.-%, yet more preferably at most 1.2 wt.-%, even more preferably at most 1.0 wt.-%, most preferably at most 0.8 wt.-%, and in particular at most 0.6 wt.-%; and/or - within the range of from 0.3±0.2 wt.-%, or 0.5±0.4 wt.-%, or 0.5±0.2 wt.-%, or 0.7±0.6 wt.-%, or 0.7±0.4 wt.-%, or 0.7±0.2 wt.-%; in each case relative to the total weight of the formulation.
Clause 46: The formulation according to any of the preceding clauses, wherein the first granular composition comprises or essentially consists of - valsartan disodium salt; - optionally, one diluent; - one binder; - one or two disintegrants; and - optionally, one or two glidants.
Clause 47: The formulation according to any of the preceding clauses, wherein the first granular composition comprises or essentially consists of - valsartan disodium salt, preferably with a total weight content of from 22 to 30 wt.-%; - optionally, one diluent, preferably with a total weight content of from 17 to 34 wt.-%; - one binder, preferably with a total weight content of from 0.01 to 6.5 wt.-%, preferably 2.5 to 6.5 wt.-%; - one or two disintegrants, preferably with a total weight content of from 0.01 to 15 wt.-%, more preferably 4.0 to 15 wt.-%; and - optionally, one or two glidants, preferably with a total weight content of from 0.5 to 2.0 wt.-%; in each case relative to the total weight of the formulation.
Clause 48: The formulation according to any of the preceding clauses, wherein the first granular composition comprises or essentially consists of - valsartan disodium salt, preferably with a total weight content of from 22 to 30 wt.-%; - optionally, microcrystalline cellulose or mannitol; preferably with a total weight content of from 17 to 34 wt.-%; - polyvinylpyrrolidone or hydroxypropyl cellulose (preferably not low substituted hydroxypropyl cellulose); preferably with a total weight content of from 0.01 to 6.5 wt.-%, preferably 2.5 to 6.5 wt.-%; - low substituted hydroxypropyl cellulose and/or crospovidone; preferably with a total weight content of from 0.01 to 15 wt.-%, more preferably 4.0 to 15 wt.-%; and - optionally, talc and/or colloidal silica; preferably with a total weight content of from 0.5 to 2.0 wt.-%; in each case relative to the total weight of the formulation.
Clause 49: The formulation according to any of the preceding clauses, wherein the second composition is a second granular composition obtained by dry granulation or hot-melt granulation, preferably dry granulation.
Clause 50: The formulation according to any of clauses 1 to 48, wherein the second composition is a second granular composition obtained by wet granulation.
Clause 51: The formulation according to any of the preceding clauses, wherein the second composition comprises one or more diluents, one or more binders, one or more disintegrants, one or more glidants, one or more lubricants, and/or mixtures thereof.
Clause 52: The formulation according to any of the preceding clauses, wherein the second composition comprises one or more disintegrants.
Clause 53: The formulation according to clause 51 or 52, wherein at least one of the one or more disintegrants comprised in the second composition is the same as one of the one or more disintegrants comprised in the first granular composition.
Clause 54: The formulation according to any of the preceding clauses, wherein the second composition does not comprise hydroxypropyl cellulose (preferably not low substituted).
Clause 55: The formulation according to any of the preceding clauses, wherein the second composition comprises crospovidone.
Clause 56: The formulation according to clause 55, wherein the total weight content of the crospovidone in the second composition is - at least 0.5 wt.-%, preferably at least 1.0 wt.-%, more preferably at least 1.5 wt.-%, still more preferably at least 2.0 wt.-%, yet more preferably at least 2.5 wt.-%, even more preferably at least 3.0 wt.-%, most preferably at least 3.5 wt.-%, and in particular at least 4.0 wt.-%; - at most 20 wt.-%, preferably at most 15 wt.-%, more preferably at most 12 wt.-%, still more preferably at most 10 wt.-%, yet more preferably at most 8.0 wt.-%, even more preferably at most 6.0 wt.-%, most preferably at most 5.0 wt.-%, and in particular at most 4.0 wt.-%; and/or - within the range of from 1.2±0.5 wt.-%, or 1.7±1.0 wt.-%, or 1.7±0.5 wt.-%, or 2.7±2.0 wt.-%, or 2.7±1.0 wt.-%, or 2.7±0.5 wt.-%, or 3.7±3.0 wt.-%, or 3.7±2.0 wt.-%, or 3.7±1.0 wt.-%, or 3.7±0.5 wt.-%, or 4.7±4.0 wt.-%, or 4.7±3.0 wt.-%, or 4.7±2.0 wt.-%, or 4.7±1.0 wt.-%, or 4.7±0.5 wt.-%; in each case relative to the total weight of the formulation.
Clause 57: The formulation according to any of the preceding clauses, wherein the first granular composition and the second composition comprise crospovidone.
Clause 58: The formulation according to clause 57, wherein the total weight content of the crospovidone in the formulation is - at least 2.0 wt.-%, preferably at least 3.0 wt.-%, more preferably at least 4.0 wt.-%, still more preferably at least 5.0 wt.-%, yet more preferably at least 6.0 wt.-%, even more preferably at least 7.0 wt.-%, most preferably at least 8.0 wt.-%, and in particular at least 9.0 wt.-%; - at most 17 wt.-%, preferably at most 15 wt.-%, more preferably at most 13 wt.-%, still more preferably at most 11 wt.-%, yet more preferably at most 9.0 wt.-%, even more preferably at most 7.0 wt.-%, most preferably at most 5.0 wt.-%, and in particular at most 3.0 wt.-%; and/or - within the range of from 2.0±0.5 wt.-%, or 2.5±1.0 wt.-%, or 2.5±0.5 wt.-%, or 3.0±1.5 wt.-%, or 3.0±1.0 wt.-%, or 3.0±0.5 wt.-%, or 5.0±3.5 wt.-%, or 5.0±1.5 wt.-%, or 5.0±1.0 wt.-%, or 5.0±0.5 wt.-%, or 7.0±5.5 wt.-%, or 7.0±3.5 wt.-%, or 7.0±1.5 wt.-%, or 7.0±1.0 wt.-%, or 7.0±0.5 wt.-%, or 9.0±7.5 wt.-%, or 9.0±5.5 wt.-%, or 9.0±3.5 wt.-%, or 9.0±1.5 wt.-%, or 9.0±1.0 wt.-%, or 9.0±0.5 wt.-%; in each case relative to the total weight of the formulation.
Clause 59: The formulation according to clause 57 or 58 wherein the total amount of crospovidone comprised in the formulation is divided between the first granular composition and the second composition.
Clause 60: The formulation according to any of the preceding clauses, wherein the first granular composition and the second composition both comprise crospovidone, and wherein the weight ratio of the crospovidone comprised in the first granular composition to the crospovidone comprised in the second composition is within the range of from 3.0:1.0 to 1.0:3.0, preferably 2.5:1.0 to 1.0:2.5, more preferably 2.0:1.0 to 1.0:2.0, still more preferably 1.5:1.0 to 1.0:1.5, and yet more preferably 1.1:1.0 to 1.0:1.1.
Clause 61: The formulation according to any of the preceding clauses, wherein the second composition comprises one or more lubricants.
Clause 62: The formulation according to clause 61, wherein the one or more lubricants are independently of one another selected from the group consisting of - metal salts of C₁₂₋₂₀-fatty acids and derivatives thereof; preferably selected from magnesium stearate, calcium stearate, aluminum stearate, zinc stearate, magnesium palmitate, magnesium oleate, and sodium stearyl fumarate; - hydrogenated oils; preferably hydrogenated vegetable oil or hydrogenated castor oil; - talc; - meads wax; - spermaceti; - boric acid; - macrogols; and - mixtures thereof; preferably from magnesium stearate, calcium stearate, talc, sodium stearyl fumarate, and mixtures thereof; more preferably sodium stearyl fumarate, magnesium stearate, and mixtures thereof.
Clause 63: The formulation according to clause 61 or 62, wherein the total weight content of the one or more lubricants is - at least 0.9 wt.-%, preferably at least 1.2 wt.-%, more preferably at least 1.5 wt.-%, still more preferably at least 1.8 wt.-%, yet more preferably at least 2.1 wt.-%, even more preferably at least 2.4 wt.-%, most preferably at least 2.7 wt.-%, and in particular at least 3.0 wt.-%; - at most 10 wt.-%, preferably at most 9.0 wt.-%, more preferably at most 8.0 wt.-%, still more preferably at most 7.0 wt.-%, yet more preferably at most 6.0 wt.-%, even more preferably at most 5.0 wt.-%, most preferably at most 4.0 wt.-%, and in particular at most 3.5 wt.-%; and/or - within the range of from 2.5±0.5 wt.-%, or 3.0±1.0 wt.-%, or 3.0±0.5 wt.-%, or 3.5±2.0 wt.-%, or 3.5±1.0 wt.-%, or 3.5±0.5 wt.-%; in each case relative to the total weight of the formulation.
Clause 64: The formulation according to any of the preceding clauses, wherein the second composition comprises or essentially consists of - sacubitril sodium salt; - one disintegrant; and - optionally, one lubricant.
Clause 65: The formulation according to any of the preceding clauses, wherein the second composition comprises or essentially consists of - sacubitril sodium salt, preferably with a total weight content of from 20 to 28 wt.-%; - one disintegrant, preferably with a total weight content of from 1.0 to 5.0 wt.-%; and - optionally, one lubricant, preferably with a total weight content of from 2.5 to 3.5 wt.-%; in each case relative to the total weight of the formulation.
Clause 66: The formulation according to any of the preceding clauses, wherein the second composition comprises or essentially consists of - sacubitril sodium salt, preferably with a total weight content of from 20 to 28 wt.-%; - crospovidone, preferably with a total weight content of from 1.0 to 5.0 wt.-%; and - optionally, magnesium stearate; preferably with a total weight content of from 2.5 to 3.5 wt.-%; in each case relative to the total weight of the formulation.
Clause 67: The formulation according to any of the preceding clauses, wherein the mixture comprises a third composition which comprises neither valsartan disodium salt nor sacubitril sodium salt and which is non-granular.
Clause 68: The formulation according to clause 67, wherein the third composition comprises one or more diluents, one or more binders, one or more disintegrants, one or more glidants, one or more lubricants, and/or mixtures thereof.
Clause 69: The formulation according to clause 67 or 68, wherein the third composition comprises or essentially consists of a disintegrant.
Clause 70: The formulation according to clause 69, wherein the disintegrant is crospovidone.
Clause 71: The formulation according to any of clauses 67 to 70, wherein the third composition comprises or essentially consists of a lubricant.
Clause 72: The formulation according to clause 71, wherein the lubricant is magnesium stearate.
Clause 73: The formulation according to any of the preceding clauses, which does not comprise sodium lauryl sulphate, preferably no surfactant at all.
Clause 74: The formulation according to any of the preceding clauses, which comprises one or more diluents, one or more binders, and one or more glidants; and wherein the one or more diluents, the one or more binders, and the one or more glidants are comprised in the first granular composition.
Clause 75: The formulation according to any of the preceding clauses, wherein the second composition does not comprise - sodium hydrogen carbonate; - pregelatinized starch; and/or - colloidal silica.
Clause 76: The formulation according to any of the preceding clauses, wherein the first granular composition comprises or essentially consists of - valsartan disodium salt; - optionally, one diluent; - one binder; - two disintegrants; and - optionally, two glidants.
Clause 77: The formulation according to any of the preceding clauses, wherein the second composition comprises or essentially consists of - sacubitril sodium salt; - one disintegrant; and - optionally, one lubricant.
Clause 78: The formulation according to any of the preceding clauses, which comprises or essentially consists of - valsartan disodium salt; - one diluent; - one binder; - three disintegrants; - two glidants; - sacubitril sodium salt; and - one lubricant; wherein the valsartan disodium salt, the one diluent, the one binder, two of the three disintegrants, and the two glidants are contained in the first granular composition; wherein the sacubitril sodium salt, one of the three disintegrants, and the one lubricant are contained in the second composition; and wherein the formulation does not comprise sodium lauryl sulphate, preferably no surfactant at all.
Clause 79: The formulation according to any of the preceding clauses, wherein the first granular composition has a flow time of at most 60 s, preferably at most 55 s, more preferably at most 50 s, still more preferably at most 45 s, yet more preferably at most 40 s, even more preferably at most 35 s, most preferably at most 30 s, and in particular at most 25 s; preferably determined in accordance with Ph. Eur. 2.9.16.
Clause 80: The formulation according to any of the preceding clauses, wherein the first granular composition has a repose angle of at most 70°, preferably at most 65°, more preferably at most 60°, still more preferably at most 55°, yet more preferably at most 50°, even more preferably at most 45°, most preferably at most 40°, and in particular at most 37°; preferably determined in accordance with Ph. Eur. 2.9.36.
Clause 81: The formulation according to any of the preceding clauses, wherein the first granular composition has a bulk volume of at least 1.0 ml/g, preferably at least 1.4 ml/g, more preferably at least 1.8 ml/g, still more preferably at least 2.2 ml/g, yet more preferably at least 2.6 ml/g, even more preferably at least 3.0 ml/g, most preferably at least 3.4 ml/g, and in particular at least 3.8 ml/g; preferably determined in accordance with Ph. Eur. 2.9.34.
Clause 82: The formulation according to any of the preceding clauses, wherein the first granular composition has a tapped volume of at least 0.8 ml/g, preferably at least 1.1 ml/g, more preferably at least 1.4 ml/g, still more preferably at least 1.7 ml/g, yet more preferably at least 2.0 ml/g, even more preferably at least 2.3 ml/g, most preferably at least 2.6 ml/g, and in particular at least 2.9 ml/g; preferably determined in accordance with Ph. Eur. 2.9.34.
Clause 83: A pharmaceutical dosage form comprising the pharmaceutical formulation according to any of the preceding clauses.
Clause 84: The dosage form according to clause 83, which is compacted or compressed.
Clause 85: The dosage form according to clause 83 or 84, which is for oral administration.
Clause 86: The dosage form according to any of clauses 83 to 85, which is selected from the group consisting of tablets, minitablets, microtablets, coated tablets, coated minitablets, coated microtablets, pills, powders, lozenges, sachets, soft gelatin capsules, hard gelatin capsules, and suppositories; preferably tablets.
Clause 87: The dosage form according to any of clauses 83 to 86, wherein the dosage form is a film-coated tablet.
Clause 88: The dosage form according to any of clauses 83 to 87, wherein the dose of valsartan disodium salt is - at least 12.9 mg, preferably at least 25.7 mg, more preferably at least 38.6 mg, still more preferably at least 51.4 mg, yet more preferably at least 64.3 mg, even more preferably at least 77.1 mg, most preferably at least 90.0 mg, and in particular at least 128.0 mg; - at most 128 mg, preferably at most 105 mg, more preferably at most 92 mg, still more preferably at most 79 mg, yet more preferably at most 66 mg, even more preferably at most 53 mg, most preferably at most 40 mg, and in particular at most 27 mg; and/or - within the range of from 10 to 200 mg, preferably from 12 to 185 mg, more preferably from 14 to 170 mg, still more preferably from 16 to 155 mg, yet more preferably from 18 to 140 mg, even more preferably from 20 to 125 mg, most preferably from 22 to 110 mg, and in particular from 25 to 128 mg.
Clause 89: The dosage form according to any of clauses 83 to 88, wherein the dose of sacubitril sodium salt is - at least 12.2 mg, preferably at least 24.3 mg, more preferably at least 36.5 mg, still more preferably at least 48.6 mg, yet more preferably at least 60.8 mg, even more preferably at least 72.9 mg, most preferably at least 85.1 mg, and in particular at least 97.2 mg; - at most 126 mg, preferably at most 103 mg, more preferably at most 90 mg, still more preferably at most 77 mg, yet more preferably at most 64 mg, even more preferably at most 51 mg, most preferably at most 38 mg, and in particular at most 25 mg; and/or - within the range of from 10 to 200 mg, preferably from 12 to 185 mg, more preferably from 14 to 170 mg, still more preferably from 16 to 155 mg, yet more preferably from 18 to 140 mg, even more preferably from 20 to 125 mg, most preferably from 22 to 110 mg, and in particular from 24 to 100 mg.
Clause 90: The dosage form according to any of clauses 83 to 89, wherein the dose of valsartan disodium salt, expressed as equivalent weight of the valsartan free acid, and sacubitril sodium salt, expressed as equivalent weight of the sacubitril free acid, is - 25.7 mg valsartan and 24.3 mg sacubitril; - 51.4 mg valsartan and 48.6 mg sacubitril; or - 102.8 mg valsartan and 97.2 mg sacubitril.
Clause 91: The dosage form according to any of clauses 83 to 90, wherein the weight ratio of the valsartan disodium salt to the sacubitril sodium salt is within the range of from 1.5:1.0 to 1.0:1.5, preferably 1.4:1.0 to 1.0:1.4, more preferably 1.3:1.0 to 1.0:1.3, still more preferably 1.2:1.0 to 1.0:1.2, and yet more preferably 1.1:1.0 to 1.0:1.1.
Clause 92: The dosage form according to any of clauses 83 to 91 for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supraventricular and ventricular arrhythmias, atrial fibrillation, atrial flutter, detrimental vascular remodeling, myocardial infarction and its sequelae, atherosclerosis, angina (whether unstable or stable), renal insufficiency (diabetic and non-diabetic), heart failure, angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.
Clause 93: A process for the preparation of a pharmaceutical formulation according to any of clauses 1 to 92, which involves granulation, preferably wet granulation.
Clause 94: The process according to clause 93, wherein the first granular composition is prepared by wet granulation.
Clause 95: The process according to clause 93 or 94, wherein the second composition is a powder.
Clause 96: The process according to clause 93 or 94, wherein the second composition is a second granular composition prepared by dry granulation or hot-melt granulation, preferably dry granulation.
Clause 97: The process according to clause 93 or 94, wherein the second composition is a second granular composition prepared by wet granulation.
Clause 98: The process according to any of clauses 93 to 97, which comprises the steps of: (a) providing a granulation liquid comprising - (i) valsartan disodium salt or (ii) valsartan free acid and sodium hydroxide; - one or more solvents; and - optionally, one or more binders; (b) providing a granulation mixture comprising one or more diluents, one or more disintegrants, one or more glidants, and/or one or more binders; (c) wet granulating the granulation mixture provided in step (b) with the granulation liquid provided in step (a) thereby obtaining a first granular composition; and (d) mixing the first granular composition obtained in step (c) with a second composition comprising sacubitril sodium; preferably wherein the second composition is non-granular or granular; thereby obtaining a compression mixture which comprises the first granular composition obtained in step (c) and the second composition comprising the sacubitril sodium.
Clause 99: The process according to clause 98, wherein in step (d) the second composition is a non-granular powder mixture.
Clause 100: The process according to clause 98 or 99, wherein in step (d) the second composition is a non-granular composition (powder mixture) that additionally comprises optionally one or more disintegrants, and optionally one or more lubricants, such that the obtained compression mixture comprises the first granular composition obtained in step (c) and the second composition comprising the sacubitril sodium, the optionally present one or more disintegrants, and the optionally present one or more lubricants.
Clause 101: The process according to clause 98, wherein in step (d) the second composition is a second granular composition, which has preferably been obtained by dry granulation, hot-melt granulation, or wet granulation.
Clause 102: The process according to clause 98 or 101, wherein in step (d) the second composition is a second granular composition that additionally comprises optionally one or more disintegrants, and optionally one or more lubricants, such that the obtained compression mixture comprises the first granular composition obtained in step (c) and the second granular composition comprising the sacubitril sodium, the optionally present one or more disintegrants, and the optionally present one or more lubricants.
Clause 103: The process according to step 102, wherein the second granular composition has been obtained by dry granulation or hot-melt granulation, preferably dry granulation.
Clause 104: The process according to step 102, wherein the second granular composition has been obtained by wet granulation.
Clause 105: The process according to any of clauses 101 to 104, wherein in step (d) the first granular composition obtained in step (c) and the second granular composition are mixed with a third composition.
Clause 106: The process according to clause 105, wherein the third composition is non-granular (a powder mixture).
Clause 107: The process according to clause 105 or 106, wherein the third composition comprises neither valsartan disodium salt nor sacubitril sodium salt.
Clause 108: The process according to any of clauses 98 to 107, wherein in step (a) valsartan free acid and sodium hydroxide are provided.
Clause 109: The process according to any of clauses 98 to 108, wherein step (a) comprises the sub-steps of: (a-1) dissolving the sodium hydroxide in the one or more solvents thereby obtaining a sodium hydroxide solution; and (a-2) adding the valsartan free acid to the sodium hydroxide solution obtained in sub-step (a-1) thereby obtaining a sodium hydroxide valsartan solution.
Clause 110: The process according to clause 109, wherein the sodium hydroxide valsartan solution obtained in sub-step (a-2) has a pH value of - at least 3.5, preferably at least 4.0, more preferably at least 4.5, still more preferably at least 5.0, yet more preferably at least 5.5, even more preferably at least 6.0, most preferably at least 6.5, and in particular at least 7.0; - at most 13.5, preferably at most 13, more preferably at most 12.5, still more preferably at most 12, yet more preferably at most 11.5, even more preferably at most 11, most preferably at most 10.5, and in particular at most 10; and/or - within the range of from 3.5 to 13.5, preferably from 4.0 to 13, more preferably from 4.5 to 12.5, still more preferably from 5.0 to 12, yet more preferably from 5.5 to 11.5, even more preferably from 6.0 to 11, most preferably from 6.5 to 10.5, and in particular from 7.0 to 10.
Clause 111: The process according to any of clauses 98 to 110, wherein the molar ratio of the sodium hydroxide to the valsartan free acid provided in step (a) is within the range of from 3.5:1.0 to 0.5:1.0, preferably 3.0:1.0 to 1.0:1.0, more preferably 2.5:1.0 to 1.5:1.0, still more preferably 2.3:1.0 to 1.7:1.0, yet more preferably 2.1:1.0 to 1.9:1.0, and in particular 2:1.
Clause 112: The process according to any of clauses 98 to 111, wherein the one or more solvents provided in step (a) comprise water and/or an organic solvent.
Clause 113: The process according to any of clauses 98 to 112, wherein the one or more solvents provided in step (a) are selected from the group consisting of water, acetone, ethanol, and mixtures thereof.
Clause 114: The process according to any of clauses 98 to 113, wherein the one or more solvents provided in step (a) is water.
Clause 115: The process according to any of clauses 98 to 114, wherein the granulation liquid provided in step (a) is a solution.
Clause 116: The process according to any of clauses 98 to 115, wherein the one or more binders optionally provided in step (a) are selected from the group consisting of - cellulose ethers; preferably selected from hydroxyethyl cellulose, hydroxypropyl cellulose, and hydroxypropyl methyl cellulose; - gelatin; - polyvinylpyrrolidone; and - mixtures thereof; preferably wherein the hydroxypropyl cellulose is not a low substituted hydroxypropyl cellulose; preferably polyvinylpyrrolidone.
Clause 117: The process according to any of clauses 98 to 116, wherein the granulation mixture provided in step (b) comprises - optionally, one diluent selected from the group consisting of - mono- or oligosaccharides or their derivatives; preferably selected from lactose, e.g. lactose monohydrate, anhydrous lactose, spray dried and/or granulated lactose; sucrose, fructose, dextrates, saccharose, raffinose, trehalose, fructose, and dextrin; - sugar alcohols; preferably selected from xylitol, mannitol, maltitol, isomalt, and sorbitol; - cellulose or cellulose derivatives; preferably powdered cellulose, microcrystalline cellulose, or silicified microcrystalline cellulose; - starch or starch derivatives; preferably low moisture starch, corn starch, pregelatinized starch, or low moisture pregelatinized starch; - silicates; preferably magnesium aluminometasilicate; - salts of phosphoric acid; preferably calcium salts of phosphoric acid; preferably selected from calcium hydrogen phosphate anhydrous and calcium hydrogen phosphate hydrate; - salts of carbonic acid; preferably calcium carbonate, sodium carbonate, potassium carbonate, calcium hydrogencarbonate, sodium hydrogencarbonate, or potassium hydrogencarbonate; - salts of lactic acid; preferably calcium lactate; and - mixtures thereof; preferably from microcrystalline cellulose, saccharides (preferably selected from lactose and saccharose), sugar alcohols (preferably mannitol), and mixtures thereof; more preferably mannitol, microcrystalline cellulose and mixtures thereof; - one or two disintegrants independently of one another selected from the group consisting of - crospovidone; - cellulose or cellulose derivatives; preferably microcrystalline cellulose, low substituted hydroxypropyl cellulose, methylcellulose, sodium salts of carboxymethyl cellulose, calcium salts of carboxymethyl cellulose, cross-linked carboxymethylcellulose and salts thereof (e.g. croscarmellose sodium and/or croscarmellose calcium); - starch and starch derivatives; preferably native starch, pregelatinized starch, sodium starch glycollate, or hydroxypropyl starch; - heteroglycanes; preferably alginic acid, sodium alginate, calcium alginate, agar, or guar gum; - glucosamines; preferably chitosan; - polacrilin potassium or docusate sodium; and - mixtures thereof; preferably from microcrystalline cellulose, croscarmellose sodium, low substituted hydroxypropyl cellulose, crospovidone, and mixtures thereof; more preferably low substituted hydroxypropyl cellulose, crospovidone and mixtures thereof; - optionally, one or two glidants independently of one another selected from the group consisting of colloidal silica, talc, magnesium trisilicate, and mixtures thereof; preferably colloidal silica, talc and mixtures thereof; and - optionally, one binder selected from the group consisting of - cellulose ethers; preferably selected from hydroxyethyl cellulose, hydroxypropyl cellulose, and hydroxypropyl methyl cellulose; - gelatin; - polyvinylpyrrolidone; and - mixtures thereof; preferably wherein the hydroxypropyl cellulose is not a low substituted hydroxypropyl cellulose; preferably hydroxypropyl cellulose.
Clause 118: The process according to any of clauses 98 to 117, wherein the granulation mixture provided in step (b) comprises - optionally, microcrystalline cellulose or mannitol; - low substituted hydroxypropyl cellulose and/or crospovidone; - optionally, talc and/or colloidal silica; and - optionally, hydroxypropyl cellulose (preferably not low substituted).
Clause 119: The process according to any of clauses 98 to 118, wherein in one of the steps (a) and (b), but not in both, the one or more binders are provided.
Clause 120: The process according to any of clauses 98 to 119, wherein in step (c) the wet granulation is performed by means of a granulating device.
Clause 121: The process according to clause 120, wherein in step (c) the granulating device is a high shear mixer or a fluid bed dryer.
Clause 122: The process according to any of clauses 98 to 121, wherein step (c) comprises the sub-steps of (c-1) optionally, heating the granulation mixture provided in step (b) to an elevated temperature; preferably to a temperature within the range of from 40-43 °C, more preferably to about 40 °C; (c-2) spraying the granulation mixture provided in step (b) or the heated granulation mixture obtained in sub-step (c-1) with the granulation liquid provided in step (a) thereby obtaining wet granules; preferably at a temperature within the range of from 25-33 °C; and (c-3) drying the wet granules obtained in sub-step (c-2) at elevated temperature thereby obtaining the first granular composition; preferably within the range of from 40-43 °C.
Clause 123: The process according to any of clauses 98 to 122, wherein the first granular composition has a loss on drying value after 5 minutes at 105±2 °C of at most 7.5 wt.-%, preferably at most 7.0 wt.-%, more preferably at most 6.5 wt.-%, still more preferably at most 6.0 wt.-%, yet more preferably at most 5.5 wt.-%, even more preferably at most 5.0 wt.-%, most preferably at most 4.5 wt.-%, and in particular at most 4.0 wt.-%.
Clause 124: The process according to any of clauses 98 to 123, wherein sacubitril is provided in step (d) in the form of sacubitril sodium salt.
Clause 125: The process according to any of clauses 98 to 124, wherein in step (d) the first granular composition is mixed with - one disintegrant selected from the group consisting of - crospovidone; - cellulose or cellulose derivatives; preferably microcrystalline cellulose, low substituted hydroxypropyl cellulose, methylcellulose, sodium salts of carboxymethyl cellulose, calcium salts of carboxymethyl cellulose, cross-linked carboxymethylcellulose and salts thereof (e.g. croscarmellose sodium and/or croscarmellose calcium); - starch and starch derivatives; preferably native starch, pregelatinized starch, sodium starch glycollate, or hydroxypropyl starch; - heteroglycanes; preferably alginic acid, sodium alginate, calcium alginate, agar, or guar gum; - glucosamines; preferably chitosan; - polacrilin potassium or docusate sodium; and - mixtures thereof; preferably from microcrystalline cellulose, croscarmellose sodium, low substituted hydroxypropyl cellulose, crospovidone, and mixtures thereof; more preferably crospovidone; and - optionally, one lubricant selected from the group consisting of - metal salts of C₁₂₋₂₀-fatty acids and derivatives thereof; preferably selected from magnesium stearate, calcium stearate, aluminum stearate, zinc stearate, magnesium palmitate, magnesium oleate, and sodium stearyl fumarate; - hydrogenated oils; preferably hydrogenated vegetable oil or hydrogenated castor oil; - talc; - meads wax; - spermaceti; - boric acid; - macrogols; and - mixtures thereof; preferably from magnesium stearate, calcium stearate, talc, sodium stearyl fumarate, and mixtures thereof; more preferably sodium stearyl fumarate, magnesium stearate, and mixtures thereof.
Clause 126: The process according to any of clauses 98 to 125, wherein in step (d) the first granular composition is mixed with - crospovidone; and - optionally, magnesium stearate.
Clause 127: The process according to any of clauses 98 to 126, wherein step (d) comprises the sub-steps (d) of: (d-1) mixing the first granular composition obtained in step (c) with sacubitril sodium and optionally one or more disintegrants thereby obtaining a first compression mixture; and (d-2) optionally, mixing the first compression mixture obtained in sub-step (d-1) with one or more lubricants thereby obtaining a second compression mixture.
Clause 128: The process according to clause 127, wherein in step (d) the mixing is performed by means of a mixing device; preferably a container mixer.
Clause 129: The process according to any of clauses 98 to 128, wherein the second composition is prepared by wet granulation, by hot-melt granulation, or by dry granulation prior to mixing with the first granular composition in step (d).
Clause 130: A pharmaceutical formulation obtainable by the process according to any of clauses 98 to 129.
Clause 131: A process for the preparation of a pharmaceutical dosage form according to any of clauses 83 to 92, the process comprising the steps of: (A) providing a pharmaceutical formulation according to any of clauses 1 to 82; (B) compressing the pharmaceutical formulation provided in step (A) by means of a tablet press thereby obtaining a tablet; and (C) optionally applying a film coating to the tablet obtains in step (B).
Clause 132: The process according to clause 131 which comprises the process according to any of clauses 98 to 129.

The following examples further illustrate the invention but are not to be construed as limiting its scope.

The examples are representative of the highest dose tablets. The composition of the lower dose tablets is proportional to the highest dose.

### Constituents of pharmaceutical formulations and pharmaceutical dosage forms:

The following formulations 1 to 10 were prepared.

Formulations 1 to 7 relate to mixtures of a first granular composition made by wet granulation (fluid bed granulation) and comprising valsartan disodium salt and a second non-granular composition comprising sacubitril sodium salt. The first granular composition forms an intragranular phase and the second non-granular composition forms an extragranular phase. The overall mixture was compacted to tablets which were subsequently film coated:

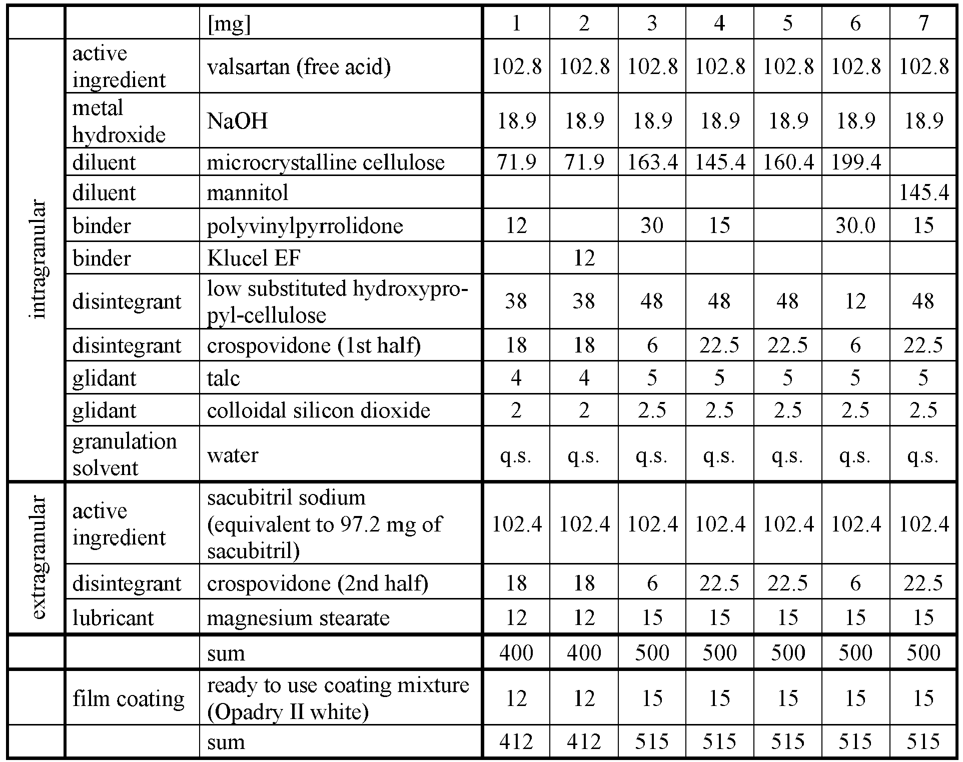

Formulations 8 to 10 relate to mixtures of a first granular composition made by wet granulation (fluid bed granulation) and comprising valsartan disodium salt, a second granular composition made by granulation (example 8 dry granulation by roller compaction; examples 9 and 10 wet granulation by fluid bed granulation) and comprising sacubitril sodium salt, and a third non-granular composition comprising neither valsartan disodium salt nor sacubitril sodium salt. The first granular composition forms a first intragranular phase, the second granular composition forms a second granular phase, and the third composition forms an extragranular phase. The overall mixture was compacted to tablets which were subsequently film coated:

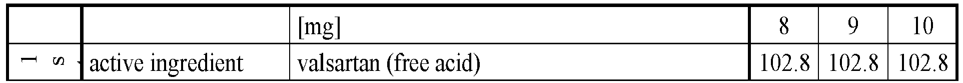

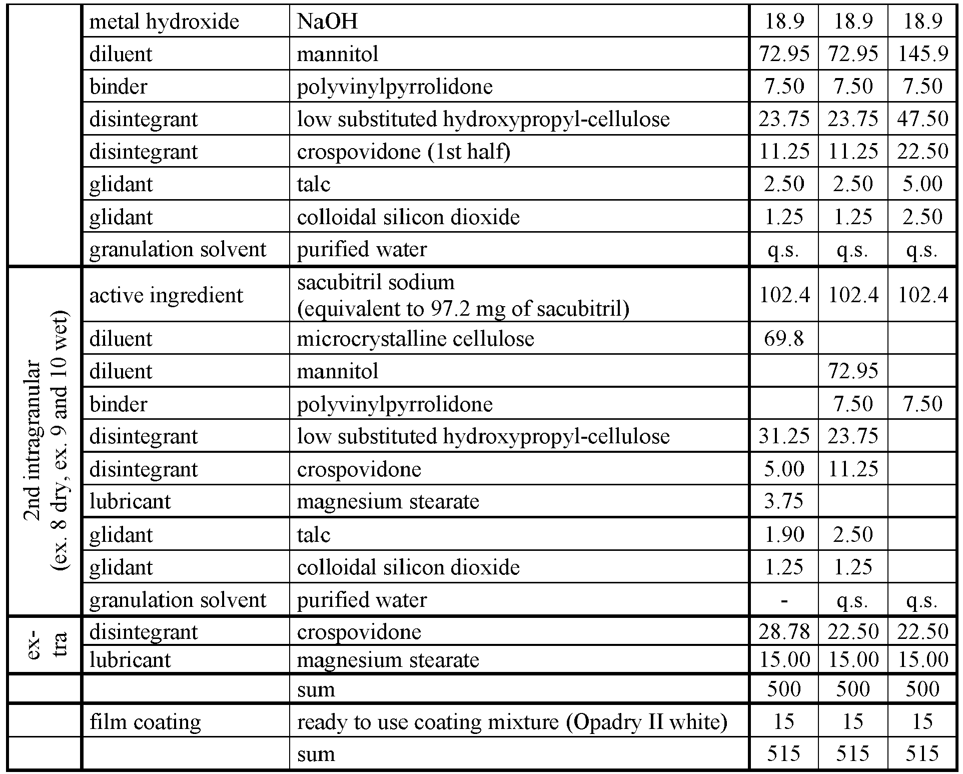

### Preparation of first granular composition comprising valsartan disodium salt by wet granulation (Examples 1 to 10):

The granulation liquid was prepared by dissolving the required amount of NaOH in an appropriate amount of water. This solution presented a clear liquid. After this, the required amount of valsartan was added during constant stirring. Valsartan dissolved completely and a clear liquid was obtained. After both NaOH and valsartan were dissolved, the pH value of the solution was measured. The pH value was between 7 and 10.

In the examples where polyvinylpyrrolidone was used, the Povidone K30 was also dissolved in the granulation liquid following the pH measurement. The obtained solution presented a clear, slightly yellow solution, which was used as the granulation liquid.

In the examples where Klucel EF was used as the binder, Klucel EF was added to the dry mixture for granulation.

The other ingredients, i.e. microcrystalline cellulose or mannitol, low substituted hydroxypropyl cellulose, talc, colloidal silicon dioxide and the first half of crospovidone were added into a fluid bed dryer.

In examples where Klucel EF was used, the Klucel EF was also added into the fluid bed dryer and not to the granulation liquid.

The contents in the fluid bed dryer were sprayed with the granulation liquid while being fluidized in the fluid bed. The granulation involved heating the granulate up to about 40 °C. After heating the granulate, was sprayed with the granulation liquid thereby starting to form the granules. The temperature of the granulate was maintained at about 25-33 °C to get optimal conditions for granule formation. After the spraying phase was finished, the granulate was dried at a temperature of about 40 °C.

The process allowed for a formation of evenly distributed granules which had significantly improved flowability compared to the dry powders or compared to granulates performed with dry granulation techniques (roller compaction).

Loss on drying (LOD) of the dry granulate was performed for 5 minutes at 105 °C. The LOD was below 4 wt.-% for optimal compression of the obtained granulate.

### Preparation of first granular composition comprising valsartan disodium salt by dry granulation (none of Example 11):

Valsartan disodium salt was dry granulated using the following composition:

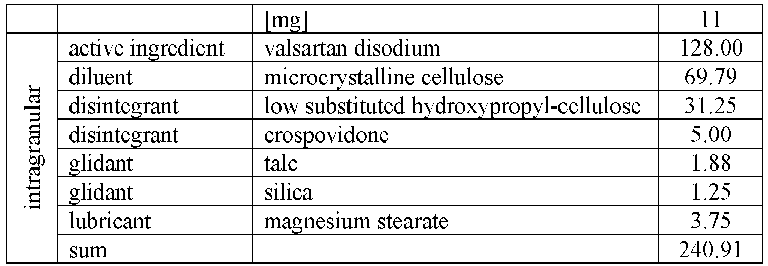

It has been surprisingly found that fluid bed granulation with valsartan disodium salt in the granulation fluid (i.e. wet granulation) leads to a faster flow time and a lower repose angle compared to dry granulation of valsartan disodium salt. The comparative data are presented in the table below:

| | | dry granulation | wet granulation |
|---|---|---|---|
| Flow time [s] | Ph. Eur. 2.9.16 | 31.1 | 20.3 |
| Repose angle [°] | Ph. Eur. 2.9.36 | 41.4 | 36.1 |
| Bulk volume [ml/g] | Ph. Eur. 2.9.34 | 2.1 | 3.9 |
| Tapped volume [ml/g] | Ph. Eur. 2.9.34 | 1.56 | 2.93 |

As demonstrated by the comparative data in the above table, fluid bed granulation with valsartan disodium salt in the granulation fluid (i.e. wet granulation) has several unexpected advantages compared to dry granulation of valsartan disodium salt.

### Preparation of second granular composition comprising sacubitril sodium salt (Examples 8 to 10):

In Example 8 the second granular composition comprising sacubitril sodium was prepared by dry granulation. All ingredients were mixed with one another and subsequently dry granulated using a roller compactor. After compaction the dry-granulate was sieved with a sieve with 0.6-2.0 mm openings.

In Examples 9 and 10 the second granular composition comprising sacubitril sodium was prepared by wet granulation. All ingredients were granulated in a fluid bed dryer. The granulating solution was prepared with water and polyvinylpyrrolidone.

### Preparation of compression mixture (Examples 1 to 10):

The compression mixture for Example 1-7 was prepared by adding sacubitril sodium and the second half of crospovidone (second non-granular composition) to the first granular composition and mixing in a container mixer for approximately 15 minutes. After mixing, the lubricant was added and the mixture was mixed in a container mixer for additional 2 minutes.

In Examples 8-10, the first granular composition and the second granular composition were joined in a container mixer and additional crospovidone was added and mixed in the container mixer for approximately 15 minutes. After mixing, the lubricant was added and the mixture was mixed in a container mixer for additional 2 minutes.

### Preparation of tablet cores (Examples 1 to 10):

The compression mixtures were compressed into oval or round tablets with a theoretical weight of 100 to 600 mg.

### Film coating of tablet cores (Examples 1 to 10):

Tablet cores of Examples 1-10 were coated in automatic coating pan with water-based film coating suspension, prepared by suspending of ready-to-use mixture, commercially available as Opadry 85F28751 II HP white. The theoretical weight of the film coated tablets is 3 % higher than the core weight.

## Claims

1. A pharmaceutical formulation comprising a mixture of
- a first granular composition comprising valsartan disodium salt; and
- a second composition comprising or essentially consisting of sacubitril sodium salt.

2. The formulation according to claim 1, wherein
- at least a portion of the valsartan disodium salt is present in amorphous form; and/or
- at least a portion of the sacubitril sodium salt is present in crystalline form.

3. The formulation according to any of the preceding claims, wherein the first granular composition is wet granulated, preferably from an aqueous starting material.

4. The formulation according to any of the preceding claims, wherein the second composition is
(i) a non-granular composition; or
(ii) a granular composition; preferably dry granulated, hot-melt granulated, or wet granulated.

5. The formulation according to any of the preceding claims, wherein the weight content of the first granular composition is
- at least 40 wt.-%, preferably at least 45 wt.-%, more preferably at least 50 wt.-%, still more preferably at least 55 wt.-%, yet more preferably at least 60 wt.-%, even more preferably at least 65 wt.-%, most preferably at least 69 wt.-%, and in particular at least 73 wt.-%;
- at most 96 wt.-%, preferably at most 92 wt.-%, more preferably at most 88 wt.-%, still more preferably at most 84 wt.-%, yet more preferably at most 80 wt.-%, even more preferably at most 76 wt.-%, most preferably at most 73 wt.-%, and in particular at most 68 wt.-%; and/or
- within the range of from 61±2.0 wt.-%, or 66±5.0 wt.-%, or 66±2.0 wt.-%, or 71±10 wt.-%, or 71±5.0 wt.-%, or 71±2.0 wt.-%, or 76±15 wt.-%, or 76±10 wt.-%, or 76±5.0 wt.-%, or 76±2.0 wt.-%; in each case relative to the total weight of the formulation.

6. The formulation according to any of the preceding claims, wherein the first granular composition comprises one or more diluents, one or more binders, one or more disintegrants, one or more glidants, one or more lubricants, and/or mixtures thereof.

7. The formulation according to any of the preceding claims, wherein the first granular composition comprises or essentially consists of
- valsartan disodium salt, preferably with a total weight content of from 22 to 30 wt.-%;
- optionally, microcrystalline cellulose or mannitol; preferably with a total weight content of from 17 to 34 wt.-%;
- polyvinylpyrrolidone or hydroxypropyl cellulose (preferably not low substituted hydroxypropyl cellulose); preferably with a total weight content of from 0.01 to 6.5 wt.-%, preferably 2.5 to 6.5 wt.-%;
- low substituted hydroxypropyl cellulose and/or crospovidone; preferably with a total weight content of 0.01 to 15 wt.-%, more preferably from 4.0 to 15 wt.-%; and
- optionally, talc and/or colloidal silica; preferably with a total weight content of from 0.5 to 2.0 wt.-%;
in each case relative to the total weight of the formulation.

8. The formulation according to any of the preceding claims, wherein the weight content of the second composition is
- at least 3.0 wt.-%, preferably at least 7.0 wt.-%, more preferably at least 11 wt.-%, still more preferably at least 15 wt.-%, yet more preferably at least 19 wt.-%, even more preferably at least 23 wt.-%, most preferably at least 27 wt.-%, and in particular at least 32 wt.-%;
- at most 60 wt.-%, preferably at most 55 wt.-%, more preferably at most 50 wt.-%, still more preferably at most 45 wt.-%, yet more preferably at most 40 wt.-%, even more preferably at most 35 wt.-%, most preferably at most 31 wt.-%, and in particular at most 27 wt.-%; and/or
- within the range of from 23±2.0 wt.-%, or 25±4.0 wt.-%, or 25±2.0 wt.-%, or 27±6.0 wt.-%, or 27±4.0 wt.-%, or 27±2.0 wt.-%, or 29±8.0 wt.-%, or 29±6.0 wt.-%, or 29±4.0 wt.-%, or 29±2.0 wt.-%, or 31±10 wt.-%, or 31±8.0 wt.-%, or 31±6.0 wt.-%, or 31±4.0 wt.-%, or 31±2.0 wt.-%, or 33±12 wt.-%, or 33±10 wt.-%, or 33±8.0 wt.-%, or 33±6.0 wt.-%, or 33±4.0 wt.-%, or 33±2.0 wt.-%; in each case relative to the total weight of the formulation.

9. The formulation according to any of the preceding claims, wherein the second composition comprises one or more diluents, one or more binders, one or more disintegrants, one or more glidants, one or more lubricants, and/or mixtures thereof.

10. The formulation according to any of the preceding claims, wherein the second composition comprises or essentially consists of
- sacubitril sodium salt, preferably with a total weight content of from 20 to 28 wt.-%;
- crospovidone, preferably with a total weight content of from 1.0 to 5.0 wt.-%; and
- optionally, magnesium stearate; preferably with a total weight content of from 2.5 to 3.5 wt.-%;
in each case relative to the total weight of the formulation.

11. A pharmaceutical dosage form comprising the pharmaceutical formulation according to any of the preceding claims.

12. The dosage form according to claim 11, wherein the dosage form is a tablet, preferably a film-coated tablet.

13. The dosage form according to claim 11 or 12 for use in the treatment or prevention of hypertension or heart failure.

14. A process for the preparation of a pharmaceutical formulation according to any of claims 1 to 10, which involves granulation, preferably wet granulation.

15. The process according to claim 14, which comprises the steps of:
(a) providing a granulation liquid comprising
- (i) valsartan disodium salt or (ii) valsartan free acid and sodium hydroxide;
- one or more solvents; and - optionally, one or more binders;
(b) providing a granulation mixture comprising one or more diluents, one or more disintegrants, one or more glidants, and/or one or more binders;
(c) wet granulating the granulation mixture provided in step (b) with the granulation liquid provided in step (a) thereby obtaining a first granular composition; and
(d) mixing the first granular composition obtained in step (c) with a second composition comprising sacubitril sodium, thereby obtaining a compression mixture which comprises the first granular composition obtained in step (c) and the second composition comprising the sacubitril sodium.
